(19) Europäisches Patentamt / European Patent Office / Office européen des brevets

(11) **EP 2 544 141 A1**

(12) **EUROPEAN PATENT APPLICATION**
published in accordance with Art. 153(4) EPC

(43) Date of publication:
**09.01.2013 Bulletin 2013/02**

(21) Application number: **11750661.8**

(22) Date of filing: **01.03.2011**

(51) Int Cl.:
*G06Q 50/00* (2012.01)        *G01N 21/27* (2006.01)
*G01N 33/483* (2006.01)

(86) International application number:
**PCT/JP2011/054657**

(87) International publication number:
**WO 2011/108551 (09.09.2011 Gazette 2011/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.03.2010 JP 2010047182**

(71) Applicant: **Olympus Corporation**
**Shibuya-ku**
**Tokyo 151-0072 (JP)**

(72) Inventor: **YAMAMOTO, Yoko**
**Tokyo 151-0072 (JP)**

(74) Representative: **Gunzelmann, Rainer**
**Wuesthoff & Wuesthoff**
**Patent- und Rechtsanwälte**
**Schweigerstraße 2**
**81541 München (DE)**

(54) **DIAGNOSTIC INFORMATION DISTRIBUTION DEVICE AND PATHOLOGY DIAGNOSIS SYSTEM**

(57)    To provide a diagnostic information distribution device and a pathology diagnosis system capable of allowing a pathologist to quickly make a diagnosis. In an embodiment of the present invention, an information distributing device (5) includes: a diagnostic area extraction processing unit (541) that extracts a diagnostic area from a stained specimen image obtained by imaging a stained specimen to be diagnosed; a diagnosis request acceptability determining unit (562) that determines a request pathologist who is requested to make a diagnosis among pathologists operating multiple pathology diagnosis devices (7); a providing information creating unit (544) that performs image processing corresponding to a predetermined observation procedure of the request pathologist with respect to image data of at least the diagnostic area; and a providing information distribution processing unit (563) that distributes diagnostic information including at least the providing information to the pathology diagnosis device (7) of the request pathologist.

FIG.3

EP 2 544 141 A1

**Description**

Field

The present invention relates to a diagnostic information distribution device and a pathology diagnosis system for distributing diagnostic information for diagnosing specimens.

Background

Various methods are widely used, for example, in pathology diagnosis, in which a sample of tissue obtained by excising an organ or performing a needle biopsy is thinly sliced into a thickness of about several micrometer to create a specimen, and the specimen is magnified and observed using a microscope in order to obtain various opinions. Among these methods, transmissive observation using an optical microscope is one of the most popular observation methods because equipment is relatively inexpensive and easy to handle, and this method has been performed through the ages. In this method, a sample harvested from a living body barely absorbs or scatters light and is nearly clear and colorless. Thus, the sample is generally stained with a dye when creating a specimen.

Various staining methods have been suggested, and the total number thereof amounts to 100 or more. In particular, regarding a pathological specimen, hematoxylin-eosin staining (hereinafter, referred to as "H&E staining") using blue-purple hematoxylin and red eosin as dyes is normally used.

In diagnosis of an H&E-stained pathological specimen, a pathologist comprehensively determines the shape and the distribution of tissues to be observed. In some cases, a method of subjecting a pathological specimen to special staining different from the H&E staining to change the color of a tissue to be observed to achieve visual enhancement is clinically used. This special staining is used, for example, when observing a tissue which is difficult to check with the H&E staining and when a tissue to be observed is deformed with the spreading of cancer and it is difficult to visually perceive the form of the tissue. However, this special staining has a problem in that the staining step takes 2 to 3 days, and it is difficult to quickly make a diagnosis. In addition, there is another problem in that the special staining increases the number of process steps to be performed by a clinical engineer. Thus, in recent years, an attempt has been made to specify a tissue within a pathological specimen without actually performing special staining by performing image processing on image data obtained by capturing the pathological specimen.

Meanwhile, in medical practice, pathologists conventionally have exchanged opinions on cases that are difficult to diagnose or are rare cases, for example, with other pathologists. Moreover, in recent years, there is a rising awareness of a so-called second opinion which consults a physician other than an attending physician for an opinion. In a consultation such as the second opinion, it is necessary to select a pathologist who is suitable for giving opinions in accordance with the case. Further, it is necessary to provide information necessary for a diagnosis to a pathologist who gives opinions.

As an example of a technique regarding a consultation between pathologists at remote sites, a technique of displaying pathologist information such as a specialized field and an experience of a consultable pathologist on a screen and transmitting image information to a pathologist selected by an attending pathologist based on the pathologist information is known (see Patent Literature 1). According to the technique of Patent Literature 1, the attending pathologist can exchange opinions with the selected pathologist by selecting a pathologist who is suitable to be requested to make a diagnosis based on the displayed pathologist information.

Citation List

Patent Literature

Patent Literature 1: Japanese Laid-open Patent Publication No. 11-195077

Summary

Technical Problem

However, there are various diagnosis modes (hereinafter referred to as "observation procedures"), which are different depending on a pathologist. For example, in diagnosis of the pathological specimen described above, generally, a pathologist images a pathological specimen to obtain a specimen image, displays the specimen image on a screen, and performs observation and diagnosis on the screen. Here, there is not only one imaging method, and in some cases, the type of image used in diagnosis is different depending on a pathologist. Thus, there is a problem in that the pathologist cannot make a diagnosis by a familiar observation procedure with respect to an image of a different type from that is usually used in diagnosis and thus cannot quickly make a diagnosis.

The present invention has been made in view of the problems of the related art, and an object of the present invention is to provide a diagnostic information distribution device and a pathology diagnosis system capable of allowing a pathologist to quickly make a diagnosis.

Solution to Problem

To solve the problem described above and achieve the object, a diagnostic information distribution device according to an aspect of the present invention is configured to be communicable with multiple pathology diagnosis devices operated by different pathologists and to distribute diagnostic information to the pathology diagnosis devices, and includes: an image acquiring unit that acquires a specimen image by imaging a diagnosis target specimen; a diagnostic area extracting unit that extracts a diagnostic area from the specimen image; a pathologist selecting unit that selects a request pathologist who is requested to make a diagnosis among the pathologists operating the multiple pathology diagnosis devices; a providing information creating unit that performs image processing corresponding to a predetermined observation procedure of the request pathologist on the image data of at least the diagnostic area so as to create providing information; and a providing information distributing unit that distributes diagnostic information including the providing information to the pathology diagnosis device of the request pathologist.

In a pathology diagnosis system according to another aspect of the present invention in which a diagnostic information distribution device and multiple pathology diagnosis devices operated by different pathologists are connected via a network, the diagnostic information distribution device includes: an image acquiring unit that acquires a specimen image by imaging a diagnosis target specimen; a diagnostic area extracting unit that extracts a diagnostic area from the specimen image; a pathologist selecting unit that selects a request pathologist who is requested to make a diagnosis among the pathologists operating the multiple pathology diagnosis devices; a providing information creating unit that performs image processing corresponding to a predetermined observation procedure of the request pathologist on the image data of at least the diagnostic area so as to create providing information; and a providing information distributing unit that distributes diagnostic information including at least the providing information to the pathology diagnosis device of the request pathologist, wherein the pathology diagnosis device includes a display processing unit that displays the providing information on a display unit.

Advantageous Effects of Invention

According to the present invention, it is possible to allow a pathologist to quickly make a diagnosis. Brief Description of Drawings
FIG. 1 is a block diagram illustrating an example of an overall configuration of a pathology diagnosis system.
FIG. 2 is a schematic diagram explaining a configuration example of a virtual slide microscope.
FIG. 3 is a block diagram illustrating an example of a functional configuration of an information distributing device.
FIG. 4 is a diagram illustrating a data flow in the pathology diagnosis system.
FIG. 5 is a flowchart illustrating the flow of processes performed by the virtual slide microscope.
FIG. 6 is a flowchart illustrating the flow of a virtual slide image generating process.
FIG. 7 is a diagram illustrating an example of a slide glass specimen.
FIG. 8 is a diagram illustrating an example of a specimen area image.
FIG. 9 is a diagram explaining a data configuration example of a focus map.
FIG. 10 is a flowchart illustrating the flow of processes performed by the information distributing device, the pathology diagnosis device, and an information integrating device.
FIG. 11 is a flowchart illustrating the flow of a diagnostic area information creating process.
FIG. 12 is a diagram illustrating an example of a diagnostic area extraction screen.
FIG. 13 is a diagram illustrating an example of an HE dye amount distribution chart.
FIG. 14 is a diagram illustrating the spectrum (absorbance value) of a cell nucleus.
FIG. 15 is a diagram illustrating an example of a diagnostic area.
FIG. 16 is a diagram explaining the principle of specifying a blood vessel area.
FIG. 17 is a schematic diagram illustrating another example of a diagnostic area.
FIG. 18 is an explanatory diagram explaining the principle of calculating the degree of irregularity of a core boundary.
FIG. 19 is an explanatory diagram explaining the principle of calculating fiber density.
FIG. 20 is an explanatory diagram explaining the principle of calculating the degree of shape irregularity.
FIG. 21 is a diagram illustrating an example of a cancer potential estimation table.
FIG. 22 is a flowchart illustrating the flow of a pathologist selecting process.
FIG. 23 is a diagram illustrating a data configuration example of a pathologist DB.

FIG. 24-1 is a diagram illustrating an example of a dataset of diagnostic organs and tissues.

FIG. 24-2 is a diagram illustrating another example of a dataset of diagnostic organs and tissues.

FIG. 25 is a diagram illustrating an example of specimen attribute information and diagnostic area information.

FIG. 26 is a diagram illustrating an example, of a request for diagnosis acceptability response displayed on the screen of a pathology diagnosis device.

FIG. 27 is a diagram illustrating an example of a notification of accessibility displayed on the screen of the pathology diagnosis device.

FIG. 28 is a diagram illustrating an example of a notification of reaching of a fixed number displayed on the screen of the pathology diagnosis device.

FIG. 29 is a diagram illustrating an example of an H-dye amount image.

FIG. 30 is a diagram illustrating an example of an E-dye amount image.

FIG. 31 is a diagram illustrating an example of a digitally stained image.

FIG. 32 is a diagram illustrating an example of a diagnosis screen.

FIG. 33 is a diagram illustrating an example of a report creation screen.

Description of Embodiments

Hereinafter, a preferred embodiment of the present invention will be described in detail with reference to the drawings. The present invention is not limited to the embodiment. In the drawings, the same portions are denoted by the same reference numerals.

Embodiment

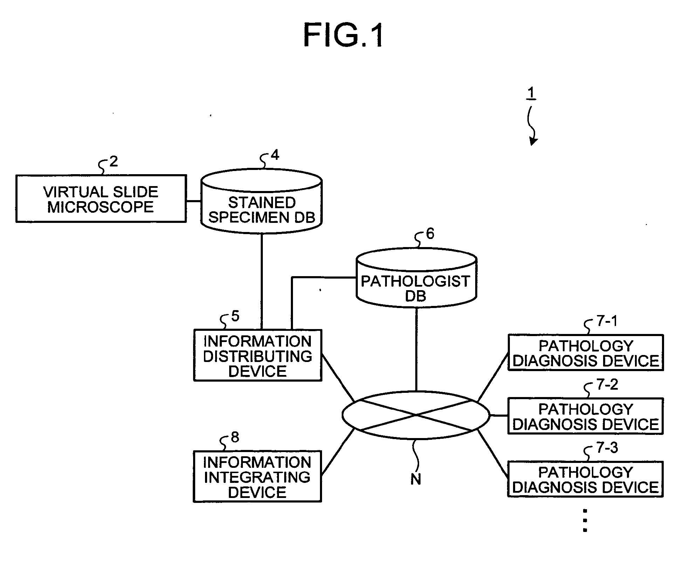

FIG. 1 is a block diagram illustrating an example of an overall configuration of a pathology diagnosis system 1 of the present embodiment. As illustrated in FIG. 1, the pathology diagnosis system 1 includes a virtual slide microscope 2 as an observing unit, a stained specimen DB 4, an information distributing device 5 as a diagnostic information distribution device, a pathologist DB 6, a pathology diagnosis device 7 (7-1, 7-2, 7-3, ...), and an information integrating device 8. In the pathology diagnosis system 1, the information distributing device 5, the pathologist DB 6, the pathology diagnosis device 7, and the information integrating device 8 are connected via a network N.

The information distributing device 5, the pathology diagnosis device 7, and the information integrating device 8 can be realized by an existing hardware configuration which includes a CPU, a main storage device such as main memory, a hard disk, an external storage device such as various storage media or the like, a communication device, an output device such as a display device or a printing device, an input device, and an interface device that connects respective units or connects an external input. For example, a general-purpose computer such as a workstation or a PC can be used as the- above devices. Moreover, one of various communication networks such as a telephone network, the Internet, a LAN, a leased line, or an intranet can be appropriately used as the network N, for example.

The virtual slide microscope 2 is a microscope device employing a virtual microscope system, and captures a specimen of an observation target to generate a virtual slide image. When a specimen is observed using a microscope device, the range (viewing range) observable at a time is mainly determined by the magnification of an objective lens. Here, a higher-resolution image can be obtained as the magnification of an objective lens increases, but the viewing range is narrowed. In order to solve this problem, a microscope system that is called a virtual microscope system in which each portion of a specimen image is captured using an objective lens having a high magnification while changing the viewing range by moving an electric stage on which a specimen is placed, and an image having high resolution and a wide field is generated by combining the individual captured partial specimen images has been conventionally known. The virtual slide image is a high-resolution and wide-field image generated by the virtual microscope system. According to the virtual microscope system, it is possible to perform observation even when a specimen is not actually present. Moreover, if the generated virtual slide image is shared to be viewable via a network, the specimen can be observed at any time and place. In recent years, the virtual microscope system has begun to be used in a consultation such as a second opinion performed between pathologists at remote sites as described above.

More specifically, the virtual slide microscope 2 of the present embodiment uses an H&E-stained living tissue specimen (hereinafter referred to as a "stained specimen") such as a pathological specimen as an observation target. Moreover, the virtual slide microscope 2 captures a multiband image of a stained specimen of an observation target to obtain a multiband virtual slide image (spectroscopic spectral image) having multi-spectrum information.

The stained specimen DB 4 is a DB (database) in which data on stained specimens of which the virtual slide images are generated by the virtual slide microscope 2 is stored. In the stained specimen DB 4, for example, a stained specimen ID which is identification information for specifying a stained specimen is registered and stored in correlation with specimen attribute information of the stained specimen, image data of a specimen image (hereinafter referred to as a "stained specimen image") of the stained specimen including a virtual slide image, diagnostic area information, providing information, and diagnosis result integration information. Hereinafter, the specimen attribute information, the image data of the stained specimen image, the diagnostic area information, the providing information, and the diagnosis result integration information will be appropriately collectively referred to as "stained specimen information."

The information distributing device 5 extracts a diagnostic area from the virtual slide image and retrieves a pathologist who is optimal for proving an opinion on the diagnostic area by referring to the pathologist information registered in the pathologist DB 6. Moreover, the information distributing device 5 modifies the virtual slide image in accordance with an observation procedure of a pathologist (hereinafter referred to as a "request pathologist"), who is finally determined as a pathologist who is requested to make a diagnosis, to create providing information and distributes the providing information to the corresponding pathology diagnosis device 7.

The pathologist DB 6 is a database in which data on pathologists is stored. In the pathologist DB 6 which is a pathologist storage unit, for example, a pathologist ID for identifying a pathologist is registered appropriately in correlation with a position, a contact address, an experience, a specialized field, a case diagnosed in the past (past case), an observation procedure, and a schedule, for example.

The pathology diagnosis device 7 is a terminal which a pathologist registered in the pathologist DB 6 uses for a diagnosis, and is provided in a medical facility where the pathologist is at work, for example. The pathology diagnosis device 7 is used for allowing a pathologist to make a diagnosis while viewing providing information or the like and send back a diagnosis result or an opinion, and is configured to display the providing information or the like distributed from the information distributing device 5, create diagnosis report information corresponding to an operation input, and transmit the diagnosis report information to the information integrating device 8.

The information integrating device 8 acquires stained specimen information of the corresponding stained specimen based on the diagnosis report information transmitted from the pathology diagnosis device 7 from the stained specimen DB 4 and creates final diagnosis result information by integrating the acquired stained specimen information.

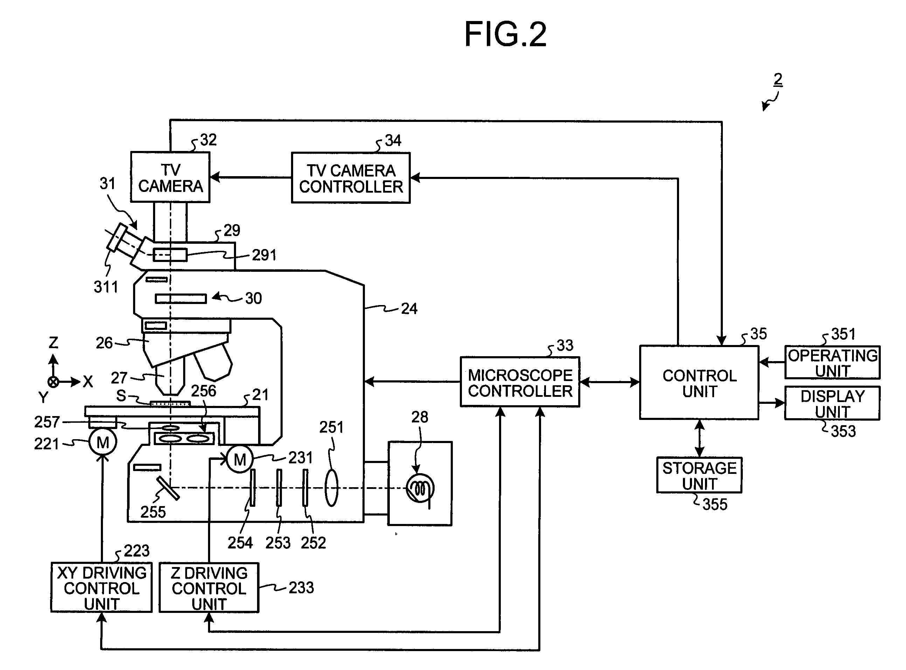

Here, the configuration of the virtual slide microscope 2 and the information distributing device 5 will be described in order. FIG. 2 is a schematic diagram explaining a configuration example of the virtual slide microscope 2. Hereinafter, an optical axis direction of an objective lens 27 illustrated in FIG. 2 will be defined as a Z direction, and a plane perpendicular to the Z direction will be defined as an XY plane.

As illustrated in FIG. 2, the virtual slide microscope 2 includes an electric stage 21 on which a stained specimen S of an observation target is placed, a microscope body 24 which has an approximately C-shape in side view and which supports the electric stage 21 and holds the objective lens 27 via a revolver 26, a light source 28 arranged at the bottom back side (the right side of FIG. 2) of the microscope body 24, and a lens barrel 29 disposed on the upper portion of the microscope body 24. Moreover, a binocular unit 31 for visually observing a specimen image of the stained specimen S and a TV camera 32 for capturing the specimen image of the stained specimen S are attached to the lens barrel 29.

The electric stage 21 is configured to be movable in the X, Y, and Z directions. Specifically, the electric stage 21 is movable in the XY plane with the aid of a motor 221 and an XY driving control unit 223 that controls the driving of the motor 221. The XY driving control unit 223 detects a predetermined origin position in the XY plane of the electric stage 21 using an XY-position origin sensor (not illustrated) under the control of a microscope controller 33 and moves an observation point on the stained specimen S by controlling the driving amount of the motor 221 from the origin position. Moreover, the XY driving control unit 223 appropriately outputs the X and Y positions of the electric stage 21 during observation to the microscope controller 33. Moreover, the electric stage 21 is movable in the Z direction with the aid of a motor 231 and a Z driving control unit 233 that controls the driving of the motor 231. The Z driving control unit 233 detects a predetermined origin position in the Z direction of the electric stage 21 using a Z-position origin sensor (not illustrated) under the control of the microscope controller 33 and moves the stained specimen S to an optional Z position within a predetermined height range to focus on the stained specimen S by controlling the driving amount of the motor 231 from the origin position. Moreover, the Z driving control unit 233 appropriately outputs the Z position of the electric stage 21 during observation to the microscope controller 33.

The revolver 26 is held to be rotatable in relation to the microscope body 24 and disposes the objective lens 27 above the stained specimen S. The objective lens 27 is attached to the revolver 26 so as to be replaceable with another objective lens having a different magnification (observation magnification) and is inserted into an optical path of observation light with the rotation of the revolver 26 so that the objective lens 27 used for observing the stained specimen S is selectively switched. In the present embodiment, the revolver 26 holds at least one objective lens (hereinafter appropriately referred to as a "low-magnification objective lens") having a relatively low magnification of 2X or 4X, for example, and at least one objective lens (hereinafter appropriately referred to as a "high-magnification objective lens") having a magnification higher than that of a low magnification objective lens of 10X, 20X, or 40X, for example, as the objective lens 27. Incidentally, the low and high magnifications are only exemplary, and at least one magnification may be higher than the other magnification.

The microscope body 24 incorporates an illumination optical system for transmissively illuminating the stained specimen S in the bottom portion thereof. The illumination optical system has a configuration in which a collector lens 251 that collects illumination light emitted from the light source 28, an illumination system filter unit 252, a field stop 253, an aperture stop 254, a folding mirror 255 that deflects the optical path of the illumination light along the optical axis of the objective lens 27, a condenser optical element unit 256, a top lens unit 257, and the like are disposed at appropriate positions along the optical path of the illumination light. The illumination light emitted from the light source 28 is irradiated onto the stained specimen S by the illumination optical system and enters the objective lens 27 as the observation light.

Moreover, the microscope body 24 incorporates a filter unit 30 in the upper portion thereof. The filter unit 30 is configured to limit a wavelength band of light imaged as the specimen image to a predetermined range and is used when the TV camera 32 captures a multiband image of the specimen image. The observation light having passed through the objective lens 27 enters the lens barrel 29 via the filter unit 30.

The filter unit 30 includes a tunable filter, a filter controller that adjusts the wavelength of light passing through the tunable filter, and the like, for example. The tunable filter is a filter which can electrically adjust the wavelength of transmission light, and one which can select a wavelength band of an optional width (hereinafter referred to as a "selected wavelength width") of 1 nm or more, for example, is used. Specifically, a commercially available product such as a liquid crystal tunable filter "VariSpec" manufactured by Cambridge Research & Instrumentation, Inc. can be appropriately used. The image data of the specimen image is obtained as a multiband image by projecting the specimen image of the stained specimen S onto an imaging device of the TV camera 32 via the filter unit 30. Here, a pixel value of each of the pixels constituting the obtained image data corresponds to the intensity of light in an optional wavelength band selected by the tunable filter, and the pixel values in the selected wavelength band are obtained with respect to the respective points of the stained specimen S. The respective points of the stained specimen S are the respective points on the projected stained specimen S corresponding to the respective pixels of the imaging device. In the following description, it is assumed that the respective points of the stained specimen S correspond to the respective pixel positions of the image data at which the respective points of the stained specimen S are obtained. The selected wavelength width of the tunable filter when capturing a multiband image may be set in advance, and an optional value can be set.

Although a configuration that uses a tunable filter has been illustrated as the configuration of the filter unit 30, the present invention is not limited to this, and an optional configuration may be employed as long as it is possible to obtain the intensity information of the light at the respective points of the stained specimen S. For example, the filter unit 30 may be configured by employing an imaging method disclosed in Japanese Laid-open Patent Publication No. 7-120324. That is, a predetermined number (for example, 16) of band-pass filters may be switched by rotating a filter wheel, and a multiband image of the stained specimen S may be captured by a field sequential method.

The lens barrel 29 incorporates a beam splitter 291 that switches the optical path of the observation light having passed through the filter unit 30 so that the observation light is guided to the binocular unit 31 or the TV camera 32. The specimen image of the stained specimen S is introduced into the binocular unit 31 by the beam splitter 291 and is visually observed by an examiner through an eye lens 311. Alternatively, the specimen image is captured by the TV camera 32. The TV camera 32 is configured to include an imaging device such as a CCD or a CMOS that images a specimen image (specifically, the viewing range of the objective lens 27), and captures the specimen image and outputs the image data of the specimen image to a control unit 35.

Moreover, the virtual slide microscope 2 includes a microscope controller 33 and a TV camera controller 34. The microscope controller 33 integratively controls the overall operations of the respective units constituting the virtual slide microscope 2 under the control of the control unit 35. For example, the microscope controller 33 rotates the revolver 26 to switch the objective lens 27 to be disposed on the optical path of the observation light, controls light modulation of the light source 28 according to the magnification of the switched objective lens 27, switches various optical elements, and instructs to move the electric stage 21 in relation to the XY driving control unit 223 or the Z driving control unit 233. In this way, the microscope controller 33 performs adjustment of the respective units of the virtual slide microscope 2 accompanied by the observation of the stained specimen S and appropriately notifies the control unit 35 of the state of each unit. The TV camera controller 34 performs ON/OFF switching of automatic gain control, gain setting, ON/OFF switching of automatic exposure control, and setting of exposure time under the control of the control unit 35 to drive the TV camera 32, and controls the capturing operation of the TV camera 32.

Moreover, the virtual slide microscope 2 includes a control unit 35 included at a proper position within the device, and the control unit 35 controls the operations of the respective units constituting the virtual slide microscope 2 to integratively control an overall operation of the virtual slide microscope 2. The control unit 35 is configured as a microcomputer and is connected to an operating unit 351, a display unit 353, a storage unit 355, and the like. The operating unit 351 is realized by various operation members such as a button switch, a slide switch, and a dial, a touch panel, a keyboard, a mouse, and the like. The display unit 353 is realized by an LCD, an EL display, or the like. The storage unit 355 is realized by various IC memories such as ROM or RAM like rewritable flash memory, a hard disk incorporated therein or connected to a data communication terminal, an information storage medium such as a CD-ROM, a reading device thereof, and the like. A program necessary for the operation of the virtual slide microscope 2, data used during execution of the program, and the like are stored in the storage unit 355.

The control unit 35 outputs an instruction on the operations of the respective units of the virtual slide microscope 2 to the microscope controller 33 and the TV camera controller 34 based on an input signal input from the operating unit 351, the states of the respective units of the virtual slide microscope 2 input from the microscope controller 33, the image data input from the TV camera 32, and the program, the data, and the like stored in the storage unit 355 to thereby integratively control the overall operation of the virtual slide microscope 2. Moreover, the virtual slide microscope 2 has an AF (automatic focus) function, and the control unit 35 performs an AF process of evaluating the contrast of an image at respective Z positions based on the image data input from the TV camera 32 and detecting a focus position (in-focus position) being focused.

The control unit 35 acquires a low-resolution image and a high-resolution image of the specimen image to generate a virtual slide image. The virtual slide image is an image generated by combining one or two or more images captured by the virtual slide microscope 2. In the following description, the virtual slide image is an image generated by combining multiple high-resolution images obtained by capturing respective portions of the stained specimen S using a high-magnification objective lens. A wide-field and high-resolution multiband image in which the entire area of the stained specimen S is photographed is referred to as the virtual slide image. That is, the control unit 35 outputs an instruction on the operations of the respective units of the virtual slide microscope 2 to acquire a low-resolution image of the specimen image. The low-resolution image is acquired as an RGB image, for example, using a low-magnification objective lens in observation of the stained specimen S. Moreover, the control unit 35 outputs an instruction on the operations of the respective units of the virtual slide microscope 2 to acquire a high-resolution image of the specimen image. The high-resolution image is acquired as a multiband image using a high-magnification objective lens in observation of the stained specimen S.

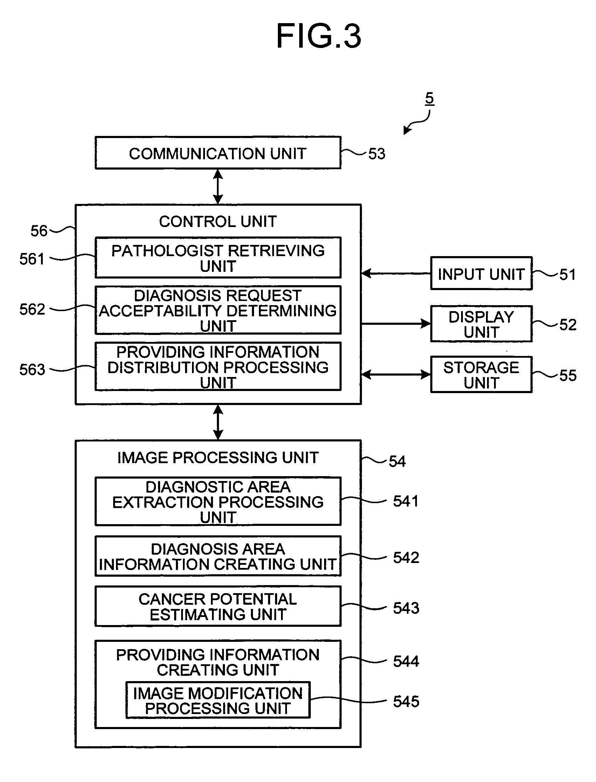

Next, the configuration of the information distributing device 5 will be described. FIG. 3 is a block diagram illustrating an example of a functional configuration of the information distributing device 5. As illustrated in FIG. 3, the information distributing device 5 includes an input unit 51, a display unit 52, a communication unit 53, an image processing unit 54, a storage unit 55, and a control unit 56 that controls the respective units of the device.

The input unit 51 is realized by a keyboard, a mouse, a touch panel, various switches, and the like, for example, and outputs an input signal corresponding to the operation input to the control unit 56. The display unit 52 is realized by a flat panel display such as an LCD or an EL display, or a display device such as a CRT display, and displays various screens in accordance with a display signal input from the control unit 56. The communication unit 53 performs data communication with an external device via the network N illustrated in FIG. 1. The communication unit 53 is realized by a modem, a TA, a jack of a communication cable, a control circuit, and the like.

The image processing unit 54 is realized by hardware such as a CPU. The image processing unit 54 includes a diagnostic area extraction processing unit 541 as a diagnostic area extracting means, a diagnosis area information creating unit 542 as a feature amount calculating means and a statistic amount calculating means, a cancer potential estimating unit 543, and a providing information creating unit 544 as a providing information creating means. The diagnostic area extraction processing unit 541 extracts a diagnostic area which is an area requiring a second opinion from the stained specimen image of a diagnosis target stained specimen. The diagnosis area information creating unit 542 calculates a predetermined feature amount of the diagnostic area extracted by the diagnostic area extraction processing unit 541 and calculates a predetermined statistic amount based on the calculated feature amount. The cancer potential estimating unit 543 estimates a cancer potential of the diagnostic area based on the statistic amount calculated by the diagnosis area information creating unit 542. The providing information creating unit 544 creates providing information corresponding to a request pathologist based on an observation procedure of the request pathologist who is retrieved by a pathologist retrieving unit 561 described later of the control unit 56 and who is determined to be requested to make a diagnosis by a diagnosis request acceptability determining unit 562 described later. The providing information creating unit 544 includes an image modification processing unit 545 as an identification image generating means and a dye amount image generating means. The image modification processing unit 545 modifies the virtual slide image of the diagnosis target stained specimen in accordance with an observation procedure of the request pathologist.

The storage unit 55 is realized by various IC memory such as ROM or RAM such as rewritable flash memory, a hard disk incorporated therein or connected to a data communication terminal, an information storage medium such as a CD-ROM, and a reading device thereof. The storage unit 55 stores temporarily or permanently a program for operating the information distributing device 5 to realize various functions of the information distributing device 5, data used during execution of the program, and the like.

The control unit 56 is realized by hardware such as a CPU. The control unit 56 outputs an instruction to respective units constituting the information distributing device 5 and transfer data to the respective units based on an input signal input from the input unit 51, the program and the data stored in the storage unit 55, or various types of information acquired from the stained specimen DB 4 and the pathologist DB 6 to thereby integratively control the overall operation of the information distributing device 5.

Moreover, the control unit 56 includes the pathologist retrieving unit 561, the diagnosis request acceptability determining unit 562, and a providing information distribution processing unit 563 as a providing information distributing means. Here, the pathologist retrieving unit 561 and the diagnosis request acceptability determining unit 562 function as a pathologist selecting means. The pathologist retrieving unit 561 retrieves pathologists who are requested to make a diagnosis by referring to the pathologist information registered in the pathologist DB 6 based on the specimen attribute information acquired from the stained specimen DB 4 and the cancer potential of the diagnostic area estimated by the cancer potential estimating unit 543, for example, to thereby select candidates (hereinafter referred to as "request candidate pathologists") of the request pathologist. The diagnosis request acceptability determining unit 562 sends a request for reply to diagnosis request to the pathology diagnosis device 7 of the request candidate pathologist retrieved and selected by the pathologist retrieving unit 561 and determines a request pathologist based on acceptability information sent from the pathology diagnosis device 7 in response to the request for reply to diagnosis request. The providing information distribution processing unit 563 distributes the providing information created by the providing information creating unit 544 to the pathology diagnosis device 7 of the request pathologist determined by the diagnosis request acceptability determining unit 562.

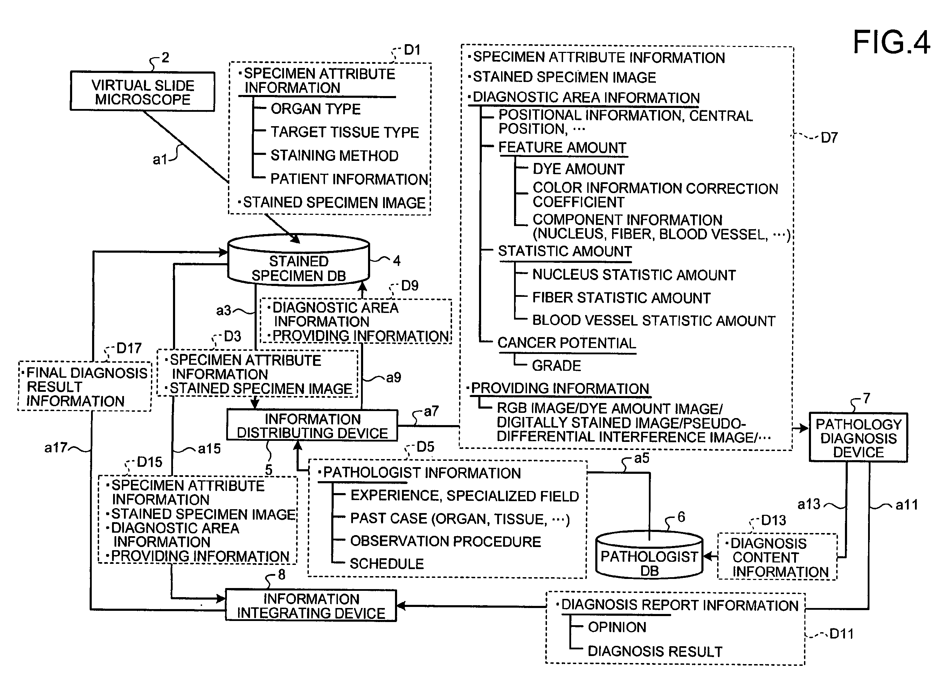

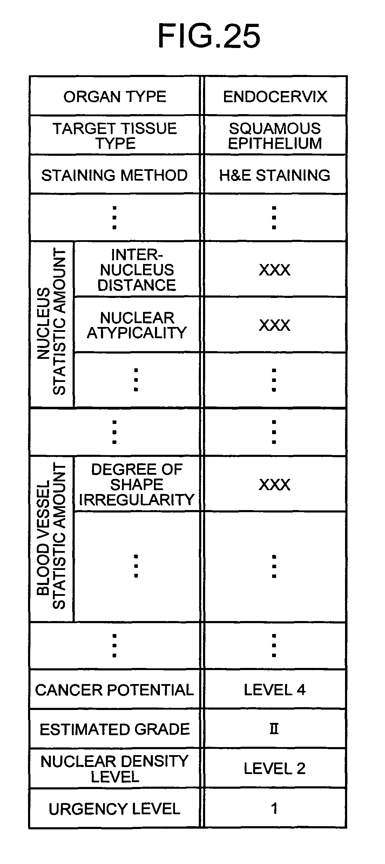

Next, the flow of data between respective devices constituting the pathology diagnosis system 1 will be described. FIG. 4 is a diagram illustrating a data flow in the pathology diagnosis system 1. In the pathology diagnosis system 1, the specimen attribute information of the stained specimen of an observation target in the virtual slide microscope 2 is acquired, and the virtual slide image of the stained specimen is generated. Moreover, as illustrated in FIG. 4, the image data (D1) of the stained specimen image including the acquired specimen attribute information and the generated virtual slide image is transmitted to the stained specimen DB 4 and registered as stained specimen information (a1). The specimen attribute information includes a type of organ, a type of target tissue, a staining method, patient information, an urgency level (not illustrated in FIG. 4, see FIG. 25), and the like.

Meanwhile, in the information distributing device 5, the specimen attribute information and the stained specimen image (D3) which are the stained specimen information of the diagnosis target stained specimen are acquired within the stained specimen information registered in the stained specimen DB 4 in this way (a3), and diagnostic area information is created based on the specimen attribute information and the stained specimen image. Here, the diagnostic area information is information on a diagnostic area extracted from the stained specimen image and is created for each of the extracted diagnostic areas. The diagnostic area information includes positional information, a central position, a feature amount, a statistic amount, a cancer potential, and the like. Examples of the feature amount include a dye amount, a color information correction coefficient, and component information on a cell nucleus, a fiber, and a blood vessel. Examples of the statistic amount include a nucleus statistic amount, a fiber statistic amount, and a blood vessel statistic amount. The cancer potential appropriately includes a grade.

After that, in the information distributing device 5, a pathologist registered in the pathologist DB 6 is retrieved. Moreover, in the information distributing device 5, the pathologist information (D5) of the request pathologist who is requested to make a diagnosis is acquired (a5), and the providing information is created based on the pathologist information. The pathologist information includes an experience, a specialized field, past cases on organs and tissues, an observation procedure, and a schedule of the corresponding pathologist. The providing information is created by modifying the image data of the stained specimen image based on the observation procedure in the pathologist information. For example, the providing information is an RGB image, a dye amount image, a digitally stained image, or a pseudo differential interference image. When there are multiple request pathologists, the providing information is individually created based on the observation procedure of the corresponding pathologist information of each of the request pathologists.

The specimen attribute information, the stained specimen image, the diagnostic area information, and the providing information (D7) of the diagnosis target stained specimen acquired or created in the information distributing device 5 are distributed to the pathology diagnosis device 7 of the request pathologist as diagnostic information (a7). When there are two or more request pathologists, data D7 (as for providing information, the providing information corresponding to the request pathologist (the pathologist of the pathology diagnosis device 7 of the distribution destination)) is distributed to the respective pathology diagnosis devices 7.

Moreover, the diagnostic area information and the providing information (D9) created in the information distributing device 5 are transmitted to the stained specimen DB 4 and are additionally registered as the stained specimen information of the diagnosis target stained specimen (a9).

In the pathology diagnosis device 7, the data D7 which is the distributed diagnostic information is displayed on a screen, for example, and presented to the request pathologist. The request pathologist makes a diagnosis while viewing the presented data D7 and inputs a diagnosis result. In the pathology diagnosis device 7, diagnosis report information is created based on the diagnosis result input by the request pathologist in this way, and the diagnosis report information (D11) is transmitted to the information integrating device 8 (a11). The diagnosis report information includes an opinion and a diagnosis result. Moreover, the diagnosis content information (D13) diagnosed by the request pathologist at that time is transmitted to the pathologist DB 6, and the pathologist information (for example, past cases or the like) of the request pathologist is updated (a13).

In the information integrating device 8, the specimen attribute information, the stained specimen image, the diagnostic area information, and the providing information (D15) which are the stained specimen information of the diagnosis target stained specimen are acquired from the stained specimen DB 4 (a15), and the data D15 is integrated with the diagnosis report information (D11) transmitted from the pathology diagnosis device 7, whereby final diagnosis result information is created. The created final diagnosis result information (D17) is transmitted to the stained specimen DB 4, and is additionally registered as the stained specimen information of the diagnosis target stained specimen (a17).

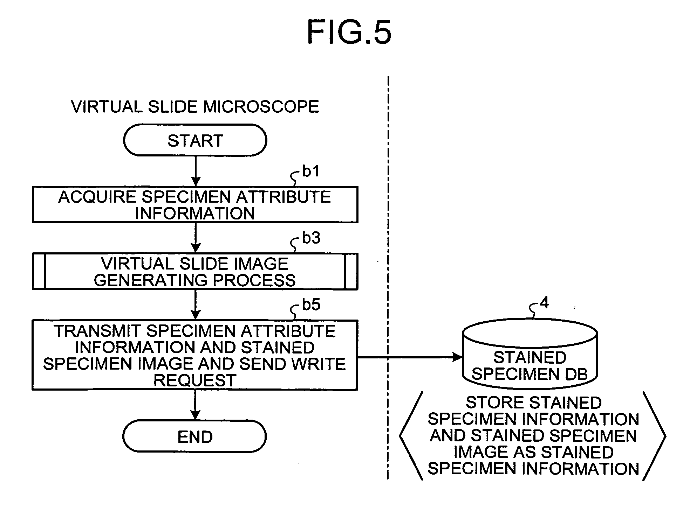

Next, the flow of processes in the pathology diagnosis system 1 will be described. First, the flow of a process in which the virtual slide image of the stained specimen is generated and is then registered and stored in the stained specimen DB 4 will be described. FIG. 5 is a flowchart illustrating the flow of processes performed by the virtual slide microscope 2.

As illustrated in FIG. 5, in the virtual slide microscope 2, first, the control unit 35 acquires the specimen attribute information of a stained specimen S in accordance with the user's operation (step b1). For example, the control unit 35 performs a process of displaying a specimen attribute input screen on the display unit 353 and sending a request to register specimen attributes. Examples of the items of the specimen attributes includes the type (organ type) of organ from which a sample is harvested, the type (target tissue type) of target tissue, the type (staining method) of staining performed on the stained specimen S, and patient information such as the name, sex, age, and previous medical history of a patient. Moreover, the control unit 35 acquires an attribute value for each of the specimen attributes input by the user's operation in response to the registration request as the specimen attribute information. Here, the control unit 35 receives an input of an urgency level for the diagnosis of the stained specimen S and acquires the same as the specimen attribute information. For example, the input of three steps of "urgent," "pressing," and "normal" is received. An urgency level of "1" is set when "urgent" is input, an urgency level of "2" is set when "pressing" is input, and an urgency level of "3" is set when "normal" is input.

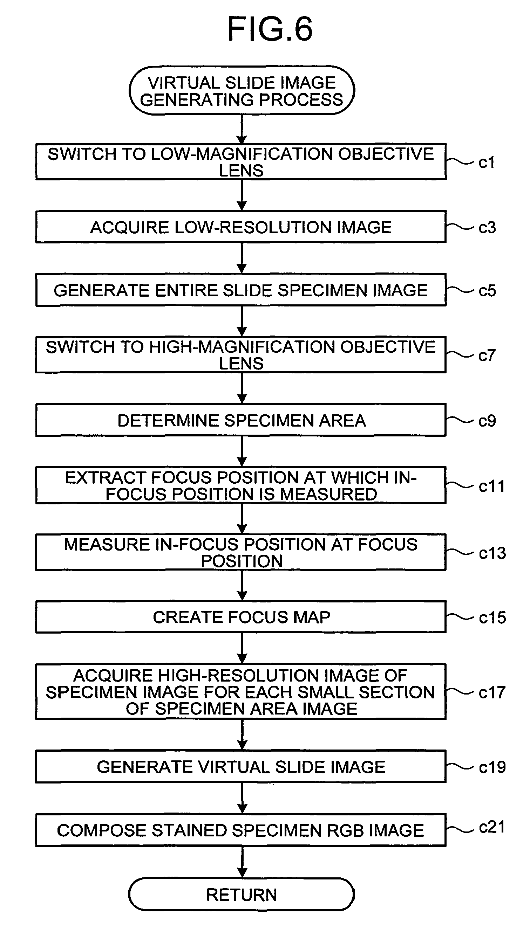

Subsequently, the control unit 35 performs a virtual slide image generating process (step b3). FIG. 6 is a flowchart illustrating the flow of the virtual slide image generating process.

As illustrated in FIG. 6, first, the control unit 35 outputs an instruction to switch the objective lens 27 used in observation of the stained specimen S to a low-magnification objective lens to the microscope controller 33 (step c1). In response to this, the microscope controller 33 rotates the revolver 26 as necessary so that the low-magnification objective lens is disposed on the optical path of the observation light.

Subsequently, the control unit 35 outputs an instruction on the operations of the respective units of the virtual slide microscope 2 to the microscope controller 33 and the TV camera controller 34 to thereby acquire a low-resolution image (RGB image) of the specimen image (step c3).

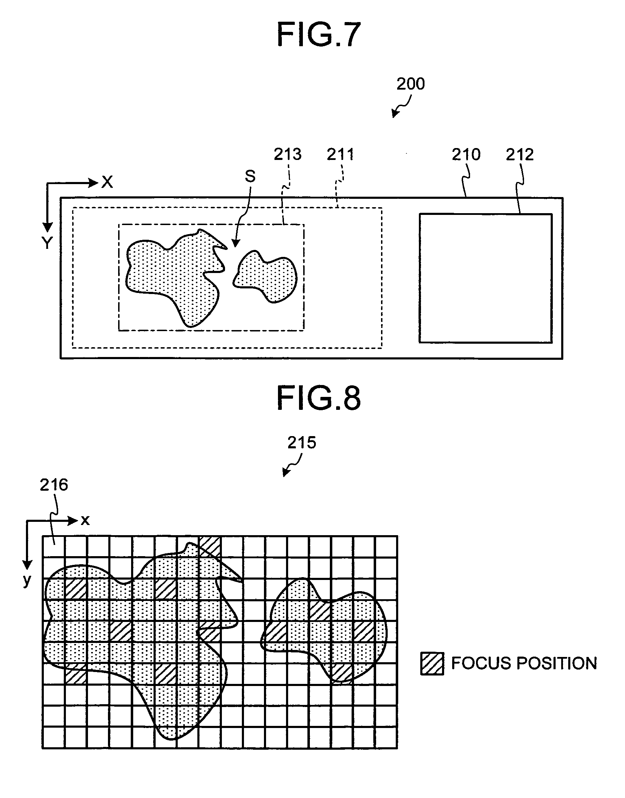

FIG. 7 is a diagram illustrating an example of a slide glass specimen 200 placed on the electric stage 21. As illustrated in FIG. 7, the stained specimen S on the electric stage 21 illustrated in FIG. 2 is actually placed on the electric stage 21 as the slide glass specimen 200 in which the stained specimen S is placed on a slide glass 210. The stained specimen S is placed within a specimen search range 211 which is a predetermined region (for example, a region of 25 mm (vertical) by 50 mm (horizontal) of the slide glass 210 on the left side of FIG. 7) on the slide glass 210. Moreover, in the slide glass 210, a label 212 that describes information on the stained specimen S placed in the specimen search range 211 is attached to a predetermined region (for example, a region on the right side of the specimen search range 211). A barcode in which the stained specimen ID allocated to the stained specimen S is coded in accordance with a predetermined standard is printed on the label 212. The barcode is read by a barcode reader (not illustrated) that constitutes the virtual slide microscope 2.

In response to the operation instruction of the control unit 35 in step c3 of FIG. 6, the virtual slide microscope 2 captures the image of the specimen search range 211 of the slide glass 210 illustrated in FIG. 7. Specifically, the virtual slide microscope 2 divides the specimen search range 211 based on the size of a viewing range (in other words, the capturing range of the TV camera 32 when a low-magnification objective lens is used in observation of the stained specimen S) determined by the magnification of the low-magnification objective lens switched in step c1 and sequentially captures the respective sections of the specimen image in the specimen search range 211 using the TV camera 32 while moving the electric stage 21 within the XY plane in accordance with the size of the divided sections. The captured image data is output to the control unit 35 as a low-resolution image of the specimen image.

Then, as illustrated in FIG. 6, the control unit 35 combines the low-resolution images of the sections acquired in step c3 to thereby generate one image in which the specimen search range 211 of FIG. 7 is photographed as the entire slide specimen image (step c5).

Subsequently, the control unit 35 outputs an instruction to switch the objective lens 27 used in observation of the stained specimen S to a high-magnification objective lens to the microscope controller 33 (step c7). In response to this, the microscope controller 33 rotates the revolver 26 so that the high-magnification objective lens is disposed on the optical path of the observation light.

Subsequently, the control unit 35 automatically extracts and determines a specimen area 213 within the specimen search range 211 of FIG. 7, in which the stained specimen S is actually placed, based on the entire slide specimen image generated in step c5 (step c9). The automatic extraction of the specimen area can be performed appropriately using the known methods. For example, the control unit 35 digitizes the pixel values of the entire slide specimen image to determine the presence of the specimen for each pixel and determines a rectangular area surrounding a range of pixels determined as the pixels in which the stained specimen S is photographed as the specimen area. The control unit 35 may receive the selection operation for the specimen area from the user via the operating unit 351 and determine the specimen area in accordance with the operation input.

Subsequently, the control unit 35 cuts the image (specimen area image) of the specimen area determined in step c9 from the entire slide specimen image and selects a position at which an in-focus position is actually measured within the specimen area image, to thereby extract a focus position (step c11).

FIG. 8 is a diagram illustrating an example of a specimen area image 215 cut from the entire slide specimen image. In FIG. 8, the image of the specimen area 213 of FIG. 7 is illustrated. First, as illustrated in FIG. 8, the control unit 35 divides the specimen area image 215 in a lattice form and forms multiple small sections. Here, the size of each small section corresponds to the size of a viewing range (that is, the capturing range of the TV camera 32 when a high-magnification objective lens is used in observation of the stained specimen S) determined by the magnification of the high-magnification objective lens switched in step c7.

Subsequently, the control unit 35 selects a small section to be used as a focus position from the multiple small sections formed. This is because the processing time increases if the in-focus position is actually measured for all small sections. Thus, the control unit 35 randomly selects a predetermined number of small sections from the small sections, for example. Alternatively, the small sections to be used as the focus position may be selected in accordance with a predetermined rule, for example, such that the selected small sections are separated by a predetermined number of small sections. Moreover, when there are a few small sections, all small sections may be selected as the focus position. Moreover, the control unit 35 calculates the central coordinates of the selected small sections in a coordinate system (x, y) of the specimen area image 215 and converts the calculated central coordinates into the coordinates of a coordinate system (X, Y) of the electric stage 21 to thereby obtain the focus position. The coordinate conversion is performed based on the magnification of the objective lens 27 used in observation of the stained specimen S or the number and the size of pixels of the imaging device constituting the TV camera 32, and can be realized by applying the known technology disclosed in Japanese Laid-open Patent Publication No. 9-281405, for example.

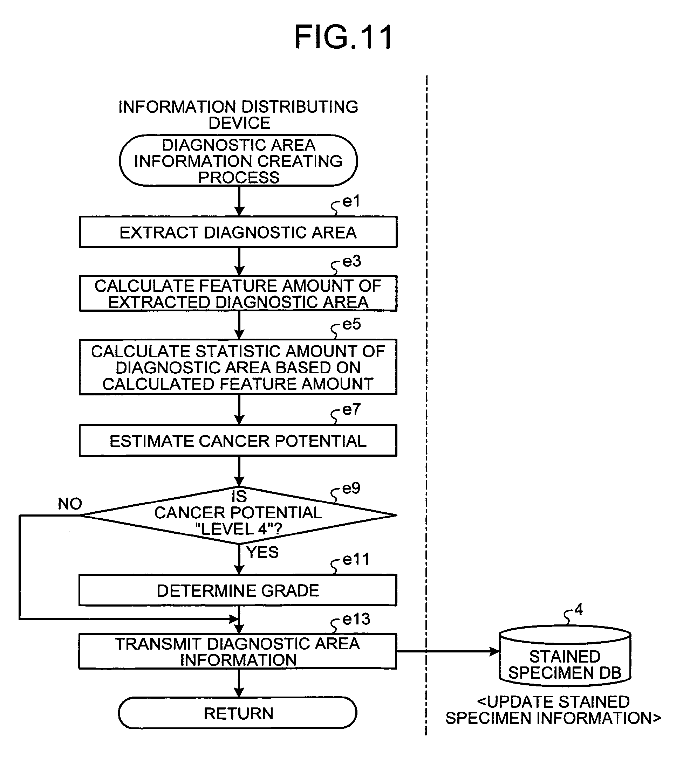

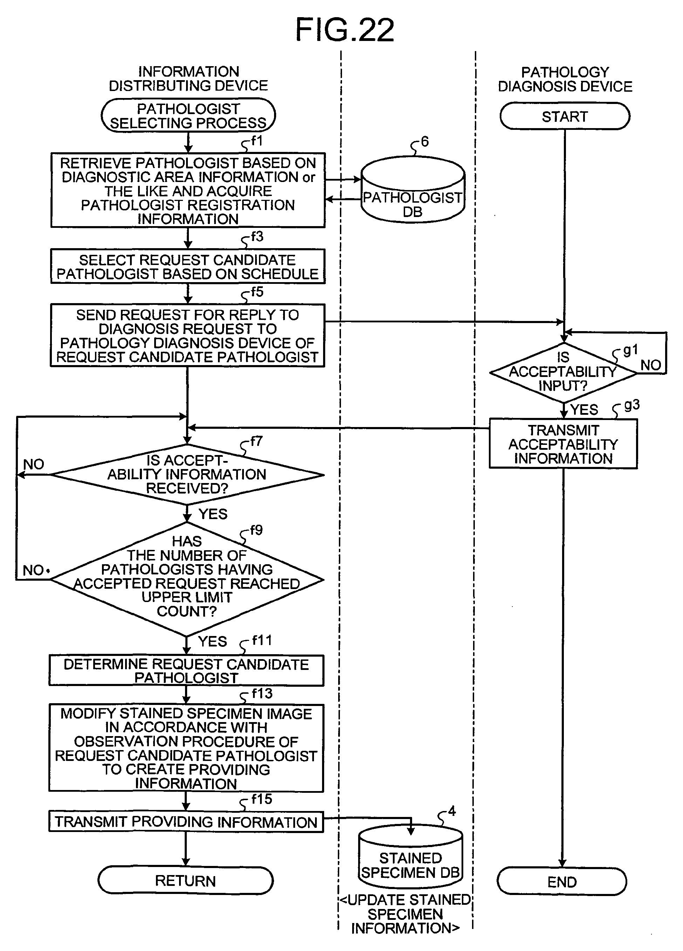

Subsequently, as illustrated in FIG. 6, the control unit 35 outputs an instruction on the operations of the respective units of the virtual slide microscope 2 to the microscope controller 33 and the TV camera controller 34 to thereby measure the in-focus position of the focus position (step c13). The control unit 35 outputs the extracted focus positions to the microscope controller 33. In response to the output, the virtual slide microscope 2 moves the electric stage 21 in the XY plane and sequentially moves the respective focus positions to the optical axis position of the objective lens 27. Moreover, the virtual slide microscope 2 receives image data at the focus positions with the aid of the TV camera 32 while moving the electric stage 21 in the Z direction at each focus position. The received image data is output to the control unit 35. The control unit 35 evaluates the contrast of the image data at the respective Z positions to measure the in-focus positions (Z positions) of the stained specimen S at the respective focus positions. In the present embodiment, at least two focus positions slightly defocused, for example, other than the in-focus position are further measured in step c13 in order to use the same when creating component information of fiber in step e3 of FIG. 11 and generating a pseudo-differential interference image in step f13 of FIG. 22, described later. For example, the positions of front and back focal points are measured. Moreover, one of the focus positions measured herein is used as a focus position F in step e3 of FIG. 11. Moreover, the two measured focus positions are used as focus positions $F_\alpha$ and $F_\beta$ in step f13 of FIG. 22. These focus positions F, $F_\alpha$, and $F_\beta$ are not limited to the front focus position and the back focus position but may be optional positions. Moreover, the number of focus positions measured in addition to the in-focus position is not limited to two, but an optional number of focus positions may be measured.

After measuring the in-focus positions at the respective focus positions in this way, subsequently, the control unit 35 creates a focus map based on the result of the measurement of the in-focus positions at the respective focus positions (step c15). Specifically, the control unit 35 interpolates the in-focus positions of small sections which are not extracted as the focus positions in step c11 at the in-focus positions of the surrounding focus positions to set the in-focus positions for all small sections to thereby create a focus map. The data of the created focus map is stored in the storage unit 355. Moreover, the control unit 35 also interpolates the front focus positions and the back focus positions of small sections which are not extracted as the focus positions at the front focus positions and the back focus positions of the surrounding focus positions to set the front focus positions and the back focus positions for all small sections. The front focus positions and the back focus positions of the respective small sections are stored in the storage unit 355 together with the focus map data.

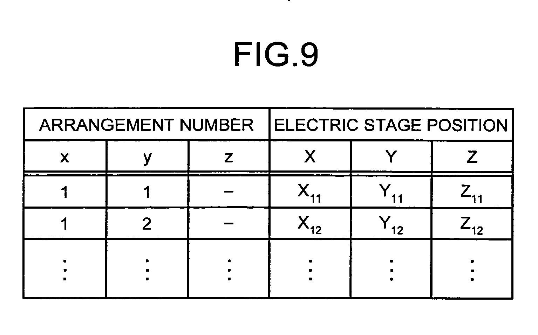

FIG. 9 is a diagram describing a data configuration example of the focus map. As illustrated in FIG. 9, the focus map is a data table in which arrangement numbers and electric stage positions are correlated with each other. The arrangement numbers indicate the individual small sections of the specimen area image 215 illustrated in FIG. 8, respectively. Specifically, the arrangement numbers indicated by x are serial numbers that are sequentially allocated to individual columns along the x direction starting from a left end, and the arrangement numbers indicated by y are serial numbers that are sequentially allocated to individual rows along the y direction starting from an uppermost stage. The arrangement numbers indicated by z are values that are set when the virtual slide image is generated as a three-dimensional image. The electric stage positions are the X, Y, and Z positions of the electric stage 21 set as the in-focus positions with respect to the small sections of the specimen area image indicated by the corresponding arrangement numbers. For example, the arrangement number of (x, y, z) = (1, 1, -) indicates a small section 216 of FIG. 8, and the X and Y positions when the central coordinates of the small section 216 in the coordinate system (x, y) are converted into the coordinates of the coordinate system (X, Y) of the electric stage 21 correspond to $X_{11}$ and $Y_{11}$, respectively. The in-focus position (Z position) set to this small section corresponds to $Z_{11}$.

Subsequently, as illustrated in FIG. 6, the control unit 35 outputs an instruction on the operations of the respective units of the virtual slide microscope 2 to the microscope controller 33 and the TV camera controller 34 while referring to the focus map as a specimen image generating means to thereby capture a multiband image of the specimen image for each of the small sections of the specimen area image to acquire a high-resolution image (step c17). The control unit 35 outputs an instruction to the microscope controller 33 such that the selected wavelength width of the tunable filter constituting the filter unit 30 is set to the width used when performing the multiband capturing. In response to this, the virtual slide microscope 2 sets the selected wavelength width of the tunable filter to the width used when performing the multiband capturing and then sequentially captures the specimen image of each of the small sections of the specimen area image at the respective in-focus positions using the TV camera 32 while moving the electric stage 21. Here, the captured image data is output to the control unit 35 as the high-resolution image of the specimen image. Moreover, the high-resolution images at the front focus position and the back focus position are also acquired based on the front focus position and the back focus position of the respective small sections.

Moreover, the control unit 35 combines the high-resolution images of the respective small sections of the specimen area image acquired in step c17 to generate one image in which the entire area of the specimen area 213 of FIG. 7 is photographed as a virtual slide image (step c19). As a result of the virtual slide image generating process described above, a wide-field and high-resolution multiband image in which the entire area of the stained specimen S is photographed is obtained. Moreover, the control unit 35 combines the high-resolution images of the respective small sections at the front focus positions to generate a virtual slide image at the front focus positions and also combines the high-resolution images of the respective small sections at the back focus positions to generate a virtual slide image at the back focus positions.

After that, as illustrated in FIG. 6, the control unit 35 composes an RGB image (stained specimen RGB image) based on multispectral information possessed by the virtual slide image (step c21). Specifically, before generating the composed stained specimen RGB image, the control unit 35 captures the background without the specimen with an illumination light illuminated to acquire a multiband image of the background (the illumination light). The multiband image of the background is set to $I_0$, the multiband image of a diagnosis target stained specimen is set to I, and spectral transmittance $t(x, \lambda)$ at each pixel position is calculated by the following equation (1). Here, the multiband image I of the diagnosis target stained specimen corresponds to the virtual slide image. Moreover, the control unit 35 also calculates the spectral transmittance $t(x, \lambda)$ at each pixel position with respect to the respective virtual slide images at the front focus position and the back focus position. Here, x is a position vector representing the pixel of a multiband image, and $\lambda$ is wavelength. Moreover, $I(x, \lambda)$ represents the pixel value at a pixel position (x) at the wavelength $\lambda$, of the multiband image I, and $I_0(x, \lambda)$ represents the pixel value at a pixel position (x) at the wavelength $\lambda$, of the multiband image Io.

$$t(x,\lambda)=\frac{I(x,\lambda)}{I_0(x,\lambda)} \qquad\qquad (1)$$

Moreover, the control unit 35 converts the spectral transmittance at the respective pixel positions obtained in this way into RGB values to thereby generate a stained specimen RGB image. When spectral transmittance at an optional pixel position (x) on a virtual slide image is set to T(x), the RGB value $G_{RGB}(X)$ is expressed by the following equation (2).

$$G_{RGB}(x) \ = \ HT(x) \qquad\qquad (2)$$

In the equation (2), H is a matrix defined by the following equation (3). This matrix H is also referred to as a system matrix, F represents spectral transmittance of the tunable filter, S represents spectral sensitivity characteristic of a camera, and E represents spectral emission characteristic of illumination.

$$H = FSE \qquad\qquad (3)$$

The control unit 35 ends the virtual slide image generating process after combining the stained specimen RGB image and returns to step b3 of FIG. 5 and proceeds to step b5.

In step b5 of FIG. 5, the control unit 35 uses the virtual slide image generated in step c19 of FIG. 6 and the stained specimen RGB image composed in step c21 as the image data of the stained specimen image, transmits the image data of the stained specimen image and the specimen attribute information acquired in step b1 of FIG. 5 to the stained specimen DB 4, and sends a write request. More specifically, the control unit 35 transmits the stained specimen ID read from the label 212 of FIG. 7 together with the specimen attribute information and the image data of the stained specimen image. In response to the request, in the stained specimen DB 4, the specimen attribute information and the image data of the stained specimen image are correlated with the stained specimen ID, and are registered and stored in the stained specimen DB 4 as the stained specimen information of the stained specimen S. In this case, the other data regarding the stained specimen S such as the data of the spectral transmittance at the respective pixel positions, calculated in the process of combining the stained specimen RGB image, for example, may be transmitted to the stained specimen DB 4 and stored so as to be included in the stained specimen information.

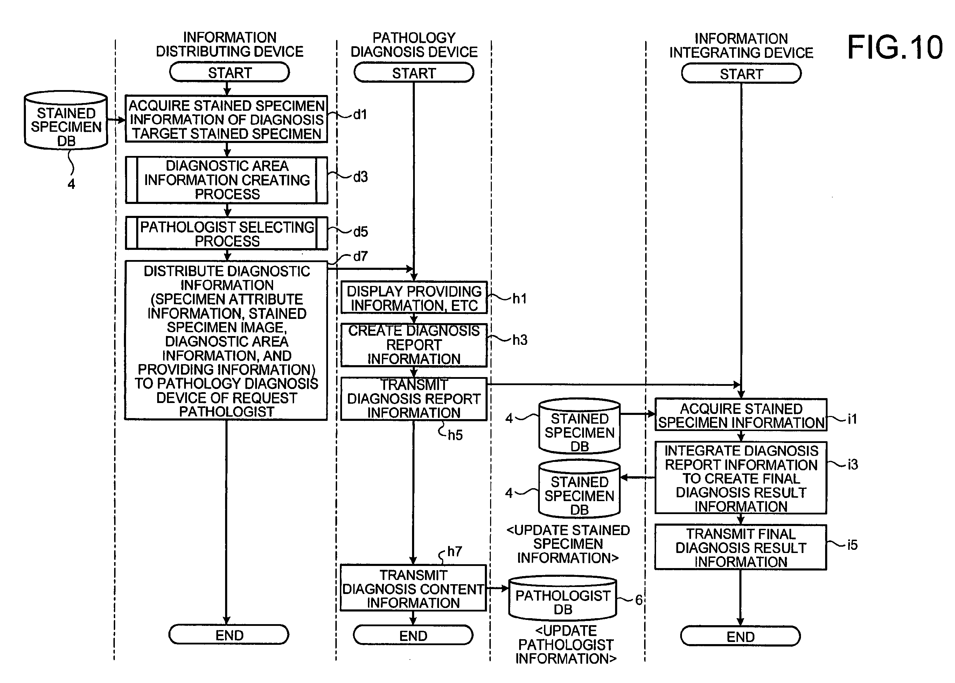

Next, the flow of processes of diagnosing the stained specimen of which the stained specimen information is registered in the stained specimen DB 4 in this way will be described. FIG. 10 is a flowchart illustrating the flow of processes performed by the information distributing device 5, the pathology diagnosis device 7, and the information integrating device 8.

As illustrated in FIG. 10, first, in the information distributing device 5, the control unit 56 acquires the stained specimen information of a diagnosis target stained specimen from the stained specimen DB 4 as an image acquiring means (step d1). Here, the diagnosis target stained specimen is selected by receiving a selection operation for the stained specimen ID from the user in advance, for example. Moreover, in step d1, the control unit 56 acquires the stained specimen information from the stained specimen DB 4 based on the stained specimen ID selected by the user. The stained specimen information acquired at that time includes at least the specimen attribute information and the image data of the stained specimen image (the virtual slide image and the stained specimen RGB image). When a diagnosis has been made at least once for the stained specimen of the selected stained specimen ID, cell nucleus identification information, diagnostic area information, providing information, and diagnosis result integration information which were created at that time are also acquired as the stained specimen information. Subsequently, the flow proceeds to a diagnostic area information creating process (step d3). FIG. 11 is a flowchart illustrating the flow of the diagnostic area information creating process.

As illustrated in FIG. 11, in the diagnostic area information creating process, first, the diagnostic area extraction processing unit 541 receives a selection operation for the diagnostic area by the user (for example, a request pathologist who is requested to make a diagnosis on the diagnosis target stained specimen) to thereby extract a diagnostic area (step e1). For example, the diagnostic area extraction processing unit 541 performs a process of displaying a diagnostic area extraction screen on the display unit 52 and sending a request to input a selection operation for the diagnostic area to the user. Moreover, the diagnostic area extraction processing unit 541 extracts the diagnostic area in accordance with the operation input of the user.

Although a case where the information distributing device 5 performs the process of receiving the selection operation for the diagnostic area to extract the diagnostic area within the stained specimen image is described, the present invention is not limited to this. For example, another device connected to the information distributing device 5 via the network N such as the pathology diagnosis device 7 may perform the process of extracting the diagnostic area and transmit the extracted positional information to the information distributing device 5. Moreover, the information distributing device 5 may perform the process subsequent to step e3 using the positional information of the diagnostic area transmitted from the other device. Moreover, when the diagnostic area information is included in the stained specimen information acquired in step d1 of FIG. 10, the diagnostic area may be specified in accordance with the diagnostic area information. That is, the diagnostic area extracted when the diagnosis target stained specimen was diagnosed in the past may be used in the process subsequent to step e3.

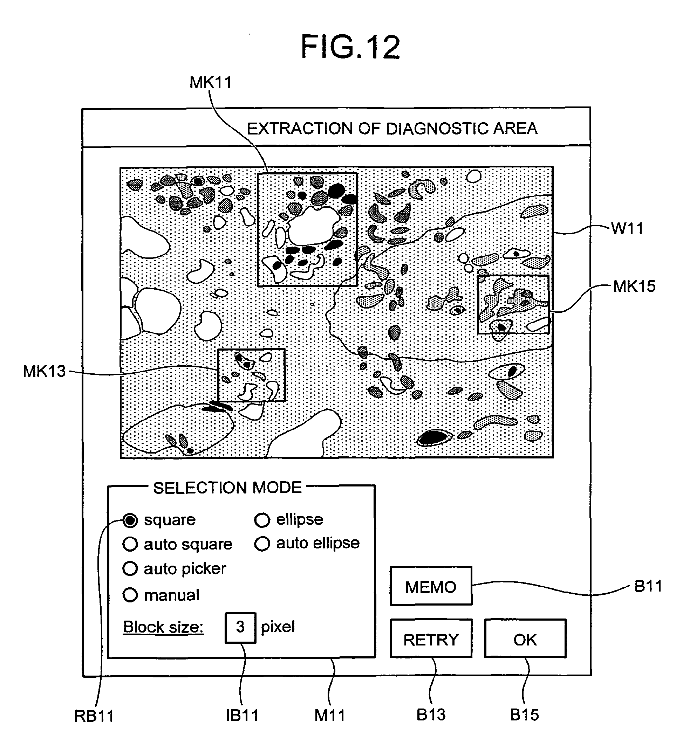

FIG. 12 is a diagram illustrating an example of the diagnostic area extraction screen. As illustrated in FIG. 12, the diagnostic area extraction screen includes a stained specimen image display portion W11. The stained specimen RGB image acquired in step c21 of FIG. 6, for example, is displayed in the stained specimen image display portion W11. Moreover, the diagnostic area extraction screen includes a selection mode menu M11, a memo button B11, a retry button B13, and an OK button B15.

In the selection mode menu M11, radio buttons RB11 are disposed so that "square," "auto square," "ellipse," "auto ellipse," "auto picker," or "manual" can be selected as a diagnostic area selection mode. The "square" is a selection mode for selecting a rectangular range on the stained specimen image display portion W11, and a rectangular range selected by the user dragging on the stained specimen image display portion W11 using a mouse constituting the input unit 51 is extracted as the diagnostic area. The "ellipse" is a selection mode for selecting an elliptical (circular) range on the stained specimen image display portion W11, and an elliptical (circular) range selected by the user dragging on the stained specimen image display portion W11 is extracted as the diagnostic area. The "auto square" is selection mode for selecting a rectangular range having a predetermined block size, and a rectangular range having a predetermined block size starting from the position clicked by the user is extracted as the diagnostic area. The block size is a block size as input in an input box IB11 described later. The "auto ellipse" is a selection mode for selecting an elliptical (circular) range inscribed in a rectangle having a predetermined block size, for example. The "auto picker" is a selection mode for automatically extracting the diagnostic area based on the pixel value at the position clicked by the user, and pixels having a brightness value similar to that of the clicked position are automatically extracted from the stained specimen RGB image, for example, and the region of the extracted pixels is used as the diagnostic area. The "manual" is a selection mode for manually selecting the diagnostic area in accordance with the user's operation, and a closed region selected by the user dragging on the stained specimen image display portion W11 starting from the position clicked by the user is extracted as the diagnostic area.

Moreover, the selection mode menu M11 includes the input box IB11 for inputting a block size, so that the user can set a desired value. For example, the block size is the size of a region designated on the stained specimen image display portion W11. As illustrated in FIG. 12, when "3" is input to the input box IB11, for example, one region has a size of 3 by 3 pixels.

The flow of the operation of selecting a region when "square" is selected as the selection mode, for example, will be described. First, the user clicks a desired position on the stained specimen image display portion W11 using a mouse constituting the input unit 51 and drags on the stained specimen image display portion W11 to thereby select a region serving as a diagnostic area, specifically a region where cancer is suspected or a region exhibiting a different aspect from the surrounding, for example. In this case, a marker (for example, a marker MK11) indicating the selected region is displayed on the stained specimen image display portion W11.

When the user wants to cancel the selection operation for the region, the user clicks on the marker (for example, the marker MK11) indicating that region to select the region and then clicks on the retry button B13. As a result, the marker MK11 is removed, and the selection operation for the region by the marker MK11 is cancelled. Moreover, when the user clicks on the marker (for example, the marker MK11) indicating the desired region to select the region and clicks on the memo button B11, the user can write a comment with respect to the region indicated by the marker MK11. For example, when the user wants to add opinions, doubtful points, and questions to the selected region, the user can write down the content thereof and can make conversations sufficiently with the request pathologist. When there are a number of regions to be used as the diagnostic area, the user can select new regions by clicking the positions of other regions on the stained specimen image display portion W11 (for example, markers MK13 and MK15). When the user wants to finalize the region selection operation, the user clicks on the OK button B15.

When the operation is finalized in this way, the diagnostic area extraction processing unit 541 extracts the region selected by the process of step e1 of FIG. 11 as the diagnostic area. For example, in the example of FIG. 12, the pixel position surrounded by the marker MK11, the pixel position surrounded by the marker MK13, and the pixel position surrounded by the marker MK15 on the stained specimen image display portion W11 are extracted as individual diagnostic areas. In this case, the diagnostic area extraction processing unit 541 calculates the central position of each of the extracted diagnostic areas. Moreover, the diagnostic area extraction processing unit 541 allocates unique diagnostic area IDs to the respective diagnostic areas in accordance with an arrangement order of the raster format and stores the positional information which is the pixel position set for the diagnostic area and the central position thereof in the storage unit 55 as the information on the diagnostic area to which the corresponding diagnostic area ID is allocated.

Subsequently, the diagnosis area information creating unit 542 calculates the feature amount of each of the diagnostic areas extracted in step e1 (step e3). Examples of the feature amount calculated herein include a dye amount, a color information correction coefficient, and component information. Hereinafter, the flow of calculating the respective feature amounts will be described in order. It is not necessary to calculate all of them as the feature amount, but at least any one of them may be calculated. Moreover, the feature amounts mentioned herein are exemplary, an additional value may be calculated based on the multispectral information possessed by the virtual slide image or the RGB value of the stained specimen RGB image and may be used as the feature amount. The value of the calculated feature amount is stored in the storage unit 55 as the information on the diagnostic area to which the corresponding diagnostic area ID is allocated.

The dye amount is a dye amount of a dye used for staining the stained specimen and is estimated based on the multispectral information possessed by the virtual slide image. The dye amount, the component information of a cell nucleus described later, and a nucleus count among the nucleus statistics are exceptionally calculated with respect to all pixel positions of the pixels constituting the virtual slide image. In the present embodiment, since the H&E stained specimen is used as an observation and diagnosis target, the dyes to be estimated include two dyes of hematoxylin (dye H) and eosin (dye E).

Here, a method of estimating quantitatively the dye amount of a staining dye used for staining points on a stained specimen based on a multiband image of the stained specimen has been conventionally known. For example, a method of estimating a dye amount and correcting color information of a stained specimen image based on the estimated dye amount is disclosed in "Color Correction of Pathological Images Based on Dye Amount Quantification" (OPTICAL REVIEW Vol. 12, No. 4 (2005), pp. 293-300). Moreover, a method of quantitatively evaluating a stained state of a specimen based on an estimated dye amount is disclosed in "Development of support systems for pathology using spectral transmittance - The quantification method of stain conditions" (Proceedings of SPIE - Image Processing, Vol. 4684, pp. 1516-1523). In this specification, the dye amount is estimated using the known technique disclosed in these documents. In the present embodiment, as described above, the respective points on the stained specimen correspond to the respective pixel positions of the obtained image data. Thus, by performing processing on each of the respective pixels of the virtual slide image, it is possible to estimate the dye amount of the dyes H and E used for staining the respective points on the stained specimen.

Hereinafter, the flow of estimation will be described briefly. First, the spectral transmittance $t(x, \lambda)$ at the respective pixel positions is calculated by the equation (1) described above. When the spectral transmittance at the respective pixel positions is stored in the stained specimen DB 4 and is acquired as the stained specimen information in step d1 of FIG. 10, the information may be used.

Regarding the spectral transmittance $t(x, \lambda)$, the Lambert-Beer law is satisfied. For example, when a stained specimen is stained with two staining dyes of the dyes H and E, the following Equation (4) is satisfied at each wavelength $\lambda$ by the Lambert-Beer law.

$$-\log t(x,\lambda) = k_H(\lambda)d_H(x) + k_E(\lambda)d_E(x) \qquad (4)$$

In the equation (4), $k_H(\lambda)$ and $k_E(\lambda)$ are coefficients unique to a substance determined depending on the wavelength $\lambda$. For example, $k_H(\lambda)$ represents a coefficient corresponding to the dye H, and $k_E(\lambda)$ represents a coefficient corresponding to the dye E. For example, the values of $k_H(\lambda)$ and $k_E(\lambda)$ are spectral characteristic values of the dyes H and E used for staining the stained specimen. Moreover, $d_H(x)$ and $d_E(x)$ correspond to the dye amounts of the dyes H and E at the respective specimen points of the stained specimen corresponding to the pixel positions $(x)$ in a multiband image. More specifically, when the dye amount of the dye H in a stained specimen stained only with the dye H is set as "1," $d_H(x)$ is obtained as a value relative to the dye amount. Similarly, when the dye amount of the dye E in a stained specimen stained only with the dye E is set as "1," $d_E(x)$ is obtained as a value relative to the dye amount. The dye amount is also referred to as density.

Here, the equation (4) is satisfied independently every wavelength $\lambda$. Moreover, the equation (4) is a linear equation of $d_H(x)$ and $d_E(x)$, and a method of solving it is generally known as multiple regression analysis. For example, by employing simultaneous equations (4) for two or more different wavelengths, they can be solved.

For example, by employing simultaneous equations for M ($M \geq 2$) wavelengths $\lambda_1$, $\lambda_2$, ..., and $\lambda_M$, they can be expressed as the following equation (5). Here, $[\ ]^t$ represents a transposed matrix, and $[\ ]^{-t}$ represents an inverse matrix.

$$\begin{bmatrix} -\log t(x,\lambda_1) \\ -\log t(x,\lambda_2) \\ \vdots \\ -\log t(x,\lambda_M) \end{bmatrix} = \begin{bmatrix} k_H(\lambda_1) & k_E(\lambda_1) \\ k_H(\lambda_2) & k_E(\lambda_2) \\ \vdots & \vdots \\ k_H(\lambda_M) & k_E(\lambda_M) \end{bmatrix} \begin{bmatrix} d_H(x) \\ d_E(x) \end{bmatrix} \qquad \cdots (5)$$

When the equation (5) is solved using least-squares estimation, the following Equation (6) is obtained. An estimation value $d\hat{}_H(x)$ of the dye amount of the dye H and an estimation value $(d)\hat{}_E(x)$ of the dye amount of the dye E are obtained. Here, "d^" represents that a symbol "^" representing the evaluation value is attached over "d."

$$\begin{bmatrix} \hat{d}_{H}(x) \\ \hat{d}_{\Xi}(x) \end{bmatrix} = \left( \begin{bmatrix} k_{H}(\lambda_{1}) & k_{E}(\lambda_{1}) \\ k_{H}(\lambda_{2}) & k_{E}(\lambda_{2}) \\ \vdots & \vdots \\ k_{H}(\lambda_{M}) & k_{E}(\lambda_{M}) \end{bmatrix}^{T} \begin{bmatrix} k_{H}(\lambda_{1}) & k_{E}(\lambda_{1}) \\ k_{H}(\lambda_{2}) & k_{E}(\lambda_{2}) \\ \vdots & \vdots \\ k_{H}(\lambda_{M}) & k_{E}(\lambda_{M}) \end{bmatrix} \right)^{-1} \begin{bmatrix} k_{H}(\lambda_{1}) & k_{E}(\lambda_{1}) \\ k_{H}(\lambda_{2}) & k_{E}(\lambda_{2}) \\ \vdots & \vdots \\ k_{H}(\lambda_{M}) & k_{E}(\lambda_{M}) \end{bmatrix}^{T} \begin{bmatrix} -\log t(x,\lambda_{1}) \\ -\log t(x,\lambda_{2}) \\ \vdots \\ -\log t(x,\lambda_{M}) \end{bmatrix} \quad \cdots \text{(6)}$$

By the equation (6), the estimation values of the dye amounts of the dyes H and E at an optional specimen point on a stained specimen are obtained.

The color information correction coefficient is the coefficient for adjusting the dye amounts of the dyes H and E estimated as described above and can be calculated using the known method. For example, the color information correction coefficient may be determined based on a dye amount distribution included in a predetermined tissue by using the method disclosed in "Color Correction of Pathological Images Based on Dye Amount Quantification." Alternatively, the color information correction coefficient may be determined based on the ratio of a coefficient representing the poorness of dye selectivity and dye amounts using the method disclosed in "Development of support systems for pathology using spectral transmittance - The quantification method of stain conditions."

The component information is one which sets a region of a predetermined component such as a cell nucleus, an elastic fiber, or a blood vessel within a diagnostic area and is created using identification pixel conditions for each component. Hereinafter, although a method of creating component information on a cell nucleus, a fiber such as an elastic fiber, and a blood vessel is described, the component information may be created for other components other than these components. Moreover, the component information may not be created for all of the cell nucleus, the elastic fiber, and the blood vessel. In this case, the component information may be created for any one of the cell nucleus, the elastic fiber, and the blood vessel. For example, the user's operation is received via the input unit 51, and the component information is created for the component designated by the user.

First, a method of creating component information of a cell nucleus will be described. Regarding the cell nucleus, the component information is created for the entire area of the stained specimen image as described above. Since the dye H selectively stains the cell nucleus, it is possible to determine whether a pixel is the pixel of a cell nucleus from color information. Therefore, in a first creation method, for example, a threshold value $Th_{B/R}$ for the B/R value is set in advance as the identification pixel condition of the cell nucleus. First, the B/R value is calculated based on the RGB value at the pixel position of a diagnostic area in a stained specimen RGB image. Subsequently, threshold processing is performed on the calculated value using the threshold value $Th_{B/R}$, and it is determined whether each pixel is the pixel of the cell nucleus. Moreover, the pixels determined to be the cell nucleus are set and used as the component information of the cell nucleus.

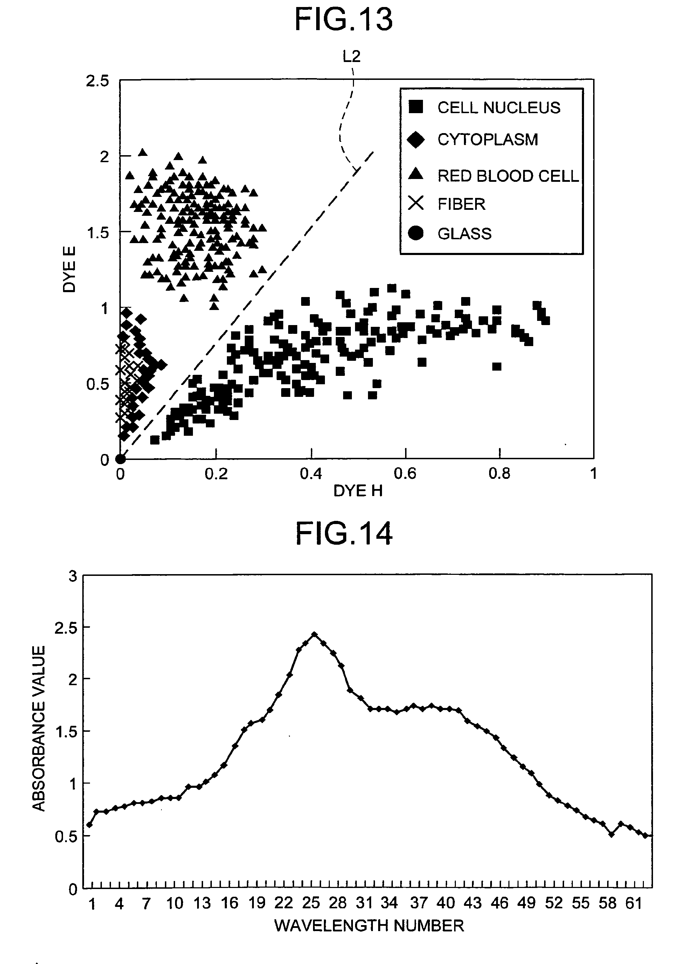

In a second creation method, a determination boundary within a dye amount space determined by the calculated (estimated) dye amounts of the dyes H and E as described above is used as the identification pixel condition of the cell nucleus, and the component information is created. FIG. 13 is a diagram illustrating an example of an HE dye amount distribution chart, representing the distribution of a cell nucleus, cytoplasm, a red blood cell, a fiber such as an elastic fiber or a collagenous fiber, and glass in an HE dye amount space in which the horizontal axis represents the dye amount of the dye H, and the vertical axis represents the dye amount of the dye E. Here, the slide glass 210 (see FIG. 7) on which the stained specimen is placed is photographed in the virtual slide image in addition to the cell nucleus, the cytoplasm, the red blood cell, and the fiber. The glass corresponds to a mapping point of the pixel in which the slide glass 210 is photographed. When the respective pixel positions of the virtual slide image are plotted in the HE dye amount space in accordance with the dye amounts of the dyes H and E, the cell nucleus pixels and the pixels other than the cell nucleus pixels can be discriminated by a determination boundary L2 indicated by a broken line in FIG. 13. In this creation method, the determination boundary is used as the identification pixel condition of the cell nucleus.

The determination boundary can be set using a determiner such as a support vector machine (SVM) or the like. For example, a determination boundary for discriminating the cell nucleus pixels and the pixels other than the cell nucleus pixels is learned using the dye content ratio R of the dyes H and E of the respective pixels in the diagnostic area as a feature amount. Moreover, the component information is created using the learned determination boundary as the identification pixel condition. The dye content ratio R of the dyes H and E is calculated by the following equation (7) based on the evaluation value $d^{\wedge}_{H}(x)$ of the dye amount of the dye H and the evaluation value $d^{\wedge}_{E}(x)$ of the dye amount of the dye E calculated by the equation (6) as described above.

$$R = \frac{\hat{d}_{E}(x)}{\hat{d}_{H}(x)} \quad \cdots \text{(7)}$$

The use of SVM makes it possible to determine the determination boundary so as to maximize the distance to a pattern closest to a boundary among the dye content ratio data (patterns) belonging to the cell nucleus, the cytoplasm, the red blood cell, the fiber, and the glass. Thus, the probability that unknown data is identified is statistically high. Therefore, by employing this creation method, it is possible to identify the cell nucleus pixels and the pixels other than the cell nucleus pixels with high accuracy.

In a third creation method, a threshold value $Th_R$ for the dye content ratio R is set in advance as the identification pixel condition of the cell nucleus. In this case, first, the dye content ratio R is calculated by the equation (7) with respect to each of the pixels constituting the diagnostic area. Moreover, threshold processing is performed on the calculated values using the threshold value $Th_R$, and the component information is created by determining whether each pixel is the pixel of the cell nucleus.

In a fourth creation method, a representative spectrum of the cell nucleus is set in advance, for example, by acquiring the spectrum of the cell nucleus pixels in an H&E stained specimen. Moreover, the component information is created using the similarity of the spectral shape as the identification pixel condition. FIG. 14 is a diagram illustrating the spectrum (absorbance value) of the cell nucleus, in which the horizontal axis represents wavelength (wavelength number), and the vertical axis represents an absorbance value. In this case, pixels of which the similarity to a spectral shape of FIG. 14 is greater than a predetermined similarity threshold value are determined as the cell nucleus pixels based on the multispectral information of the respective pixels constituting the diagnostic area in the virtual slide image.

While four creation methods for creating the component information of the cell nucleus have been described, any one of the creation methods may be employed, and pixels satisfying the respective pixel identification conditions may be extracted using a combination of multiple pixel identification conditions illustrated above to create the component information.

Next, a method of creating the component information of an elastic fiber which is one type of fiber will be described. For example, first, pixels corresponding to the elastic fiber are extracted by a learning-based determination process using SVM or the like based on the multispectral information of the respective pixels constituting a diagnostic area in a virtual slide image. Moreover, the extracted pixels are set and used as the component information of the elastic fiber. Since the elastic fiber has a phenomenon in which the spectral transmittance becomes 1.0 or higher at a certain band, the elastic fiber pixels may be extracted based on this phenomenon.

Moreover, when creating the component information of the fiber, first, a variation spectral image of the diagnostic area is generated based on the multispectral information at the respective pixel position of the pixels constituting the diagnostic area in the virtual slide image. Specifically, the front focus position or the back focus position measured in step c13 of FIG. 6 is used as the focus position F. Moreover, the spectral transmittances of the respective pixels in the diagnostic area are acquired by referring to the spectral transmittances calculated by the equation (1) in step c21 of FIG. 6 with respect to the respective pixel positions of the virtual slide image at the front focus position or the back focus position used as the focus position F. Subsequently, a variation between optional wavelengths $\lambda_1$ and $\lambda_2$ (inter-wavelength variation; an absolute value of the difference between the spectral transmittances at predetermined wavelengths) is calculated for each pixel. The wavelengths at which the variation is calculated may be selected appropriately. Moreover, a pixel value corresponding to the magnitude of the inter-wavelength variation is allocated to the respective pixels, for example, such that pixels of which the calculated inter-wavelength variation is the largest have a pixel value of "255," and pixels of which the inter-wavelength variation is zero have a pixel value of "0 (zero)." In this way, the variation spectral image is created. After that, a combination of known image processes such as smoothing, digitization, edge extraction, or morphology (expansion and contraction) is selectively performed on the created variation spectral image, whereby the fiber pixels are extracted. Moreover, the extracted pixels are set and used as the component information of the fiber.

A differential spectral image may be generated instead of the variation spectral image of the diagnostic area. In this case, the front focus position and the back focus position other than the in-focus position measured in step c13 of FIG. 6, for example, are used as the focus positions $F_\alpha$ and $F_\beta$. Moreover, the spectral transmittances of the respective pixels in the diagnostic area are acquired by referring to the spectral transmittances calculated for the respective pixel positions of the pixels of the virtual slide image at the front focus position and the back focus position. Moreover, the difference between the spectral transmittances at a predetermined wavelength $\lambda$ for the same pixels is calculated, a pixel value corresponding to the difference is allocated to the respective pixels, whereby the differential spectral image is generated. After that, a combination of known image processes is selectively performed on the created differential spectral image. In this way, the fiber pixels may be extracted and used as the component information of the fiber.

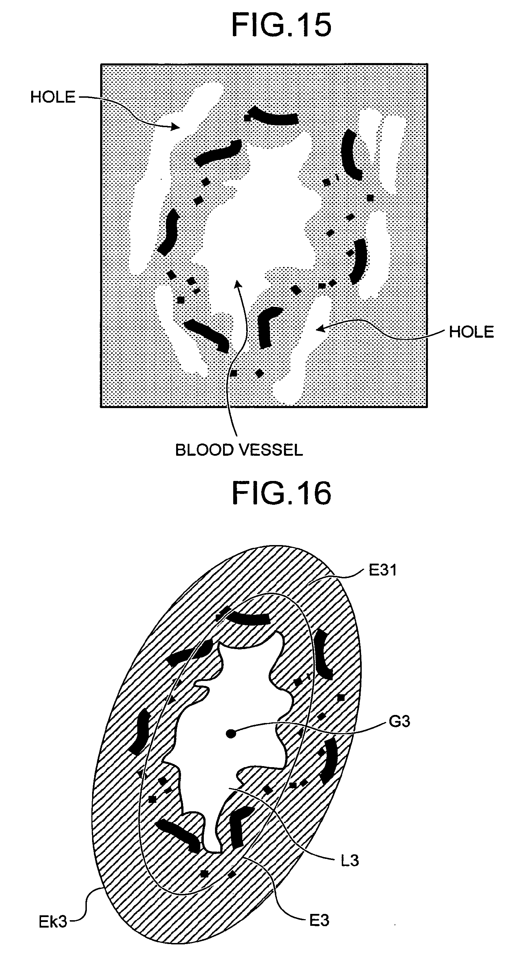

Next, a method of creating the component information of a blood vessel will be described. FIG. 15 is an example of a diagnostic area, illustrating a region in which a blood vessel is photographed at the center. When creating the component information of the blood vessel, first, a blood vessel and a hole illustrated in FIG. 15 are specified. Here, an intravascular lumen in the stained specimen and a region (hole) where a tissue around a blood vessel is not present are photographed white as illustrated in FIG. 15 and appear as regions having high-brightness pixels. Thus, high-brightness regions are extracted from the diagnostic area and are specified as blood vessel areas or hole areas.

Specifically, first, elastic fiber pixels in the diagnostic area are extracted by the same method as the method of creating the component information of the elastic fiber. Subsequently, high-brightness regions are extracted from the diagnostic area. As described above, the high-brightness regions in the diagnostic area are expected to be blood vessel areas or hole areas where no tissue is present. Thus, the image of the diagnostic areas is converted into a grayscale image based on the RGB value of the respective pixels of the diagnostic area. Moreover, threshold processing is performed on the brightness values of the respective pixels using a predetermined threshold value, and pixels of which the brightness value is the threshold value or greater are selected as the high-brightness pixels. After that, a set of connected pixels among the selected high-brightness pixels is extracted as one high-brightness region.

After the high-brightness regions are extracted in this way, a high-brightness region around which an elastic fiber is present among the extracted high-brightness regions is specified as the blood vessel area. Specifically, first, the contour, the central position, and the boundary length of the extracted high-brightness region are calculated to approximate the high-brightness region to an ellipse to set an ellipse, and a K-magnification ellipse obtained by magnifying the ellipse by K times is set. FIG. 16 is a diagram describing the principle of specifying a blood vessel area, in which a central position G3 obtained for the extracted high-brightness region L3, an ellipse E3 set by approximating the high-brightness region L3 to an ellipse, and a K-magnification ellipse Ek3 are illustrated. The K-magnification ellipse is an ellipse which has the same central position as an original ellipse and has an area K times the original ellipse. The value of K can be set appropriately. The high-brightness region L3 illustrated in FIG. 16 corresponds to a blood vessel area which is photographed white at the center of the diagnostic area illustrated in FIG. 15.

After that, the positional relation between the high-brightness region and the elastic fiber is determined. Moreover, when an elastic fiber is present closely around the contour position of the high-brightness region, the high-brightness region is specified as the blood vessel area. Specifically, it is determined whether a predetermined amount of elastic fiber or more is present in a region E31 hatched in FIG. 16 and surrounded by the contour line of the high-brightness region L3 and the K-magnification ellipse Ek3. For example, the number of elastic fiber pixels in the region E31 is measured. When the measured number of elastic fiber pixels is a predetermined threshold value or greater, it is determined that a predetermined amount of elastic fiber or more is present. Moreover, the pixels of the high-brightness region L3 in which it is determined that a predetermined amount of elastic fiber or more is present are set and used as the component information of the blood vessel.

After the feature amount is calculated for each diagnostic area in this way, as illustrated in FIG. 11, the diagnosis area information creating unit 542 calculates the statistic amount for each diagnostic area based on the calculated feature amount (step e5). Examples of the calculated statistic amount include a statistic amount (nucleus statistic amount) for a cell nucleus, a statistic amount (fiber statistic amount) for a fiber, and a statistic amount (blood vessel statistic amount) for a blood vessel. Hereinafter, the flow of calculating the respective statistic amounts will be described in order. It is not necessary to calculate all of them as the statistic amount, but at least any one of them may be calculated. Moreover, the statistic amounts mentioned herein are exemplary, and an additional value may be calculated based on the feature amount of the diagnostic area and may be used as the statistic amount. The value of the calculated statistic amount is stored in the storage unit 55 as the information on the diagnostic area to which the corresponding diagnostic area ID is allocated.

First, the nucleus statistic amount will be described. In cancer portions, it is known that cell nuclei are present at a concentrated local area. Moreover, when cancer progresses, the shape of a cell nucleus is deformed and changed as compared to a normal case. Thus, the number (nucleus count) of cell nuclei in the diagnostic area, the distance (inter-nucleus distance) between cell nuclei, and nuclear atypicality are calculated as the nucleus statistic amount, for example.

First, closed regions made up of the cell nucleus pixels (hereinafter, referred to as "nucleus pixels") are specified as individual cell nucleus areas based on the component information of the cell nucleus calculated as the feature amount. As described above, the nucleus count is measured for the entire area of the stained specimen image. Thus, the cell nucleus area is specified for the entire area of the stained specimen image.

Specifically, nucleus pixels are segmented in respective connecting components, and each of the segmented sets of pixels is specified as a cell nucleus area. The connectivity is appropriately determined using a known method, and for example, the connectivity may be determined at eight neighboring positions. Moreover, a unique label (nucleus label) NL is labeled to each connecting component, whereby respective sets of pixels for each connecting component are specified as cell nucleus areas, respectively. The labeling method may appropriately employ a known method. For example, a method of labeling the respective pixels in the order of raster scanning (the order of scanning each line in the left to right direction downwardly from the uppermost line of the diagnostic area) can be used, and nucleus labels (NL = 1, 2, 3, ..., and so on) are labeled to each connecting component in ascending order of integers.

After labeling is finished, the number (nucleus count) of cell nucleus areas in a specified stained specimen image is measured. Moreover, the nucleus count is measured for each diagnostic area.

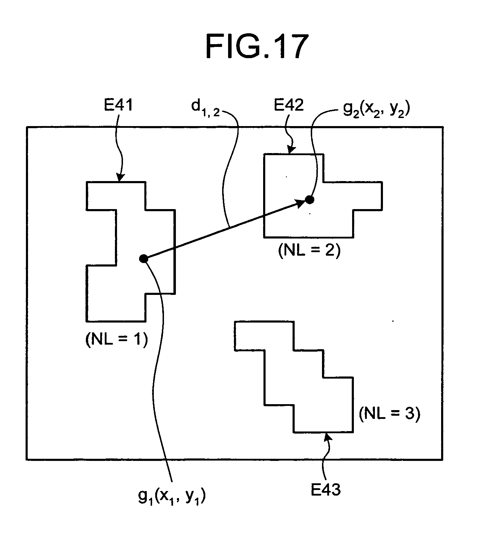

After that, processing is performed for each diagnostic area. Specifically, first, the distance between cell nucleus areas is calculated as an inter-nucleus distance. FIG. 17 is a schematic view illustrating an example of a diagnostic area, in which three cell nucleus areas E41, E42, and E43 labeled with the nucleus labels NL = 1, 2, and 3, respectively, are illustrated. In order to calculate the inter-nucleus distance, first, the central positions of the respective cell nucleus areas E41, E42, and E43 are calculated. Here, when the central position of the cell nucleus area E41 labeled with the nucleus label NL = 1 is $g_1(x_1, y_1)$, and the central position of the cell nucleus area E42 labeled with the nucleus label NL = 2 is $g_2(x_2, y_2)$, the inter-nucleus distance $d_{1,2}$ between the cell nucleus areas E41 and E42 is expressed by the following equation (8). Similarly, the inter-nucleus distance between the cell nucleus areas E41 and E43 and the inter-nucleus distance between the cell nucleus areas E42 and E43 are also calculated.

$$d_{1,2} = \sqrt{(x_1 - x_2)^2 + (y_1 - y_2)^2} \qquad \cdots (8)$$

Moreover, the level of nuclear atypicality is determined for each of the specified cell nucleus areas. In this example, the level of nuclear atypicality is determined, for example, using the size S of a cell nucleus, the degree of irregularity $\sigma$ of a core boundary, and the degree C of circular deformation as the indices indicating the degree of difference in shape as compared to a normal cell nucleus.

First, the size S of a cell nucleus is obtained by measuring the number of pixels constituting the corresponding cell nucleus area.

The degree $\sigma$ of irregularity of a core boundary is obtained by calculating a variance of straight lines connecting the pixel positions that form the contour of the cell nucleus area. Here, the cell nucleus area has a smoother contour shape as the variance decreases, and the degree $\sigma$ of irregularity of the core boundary is determined by the magnitude of the variance.

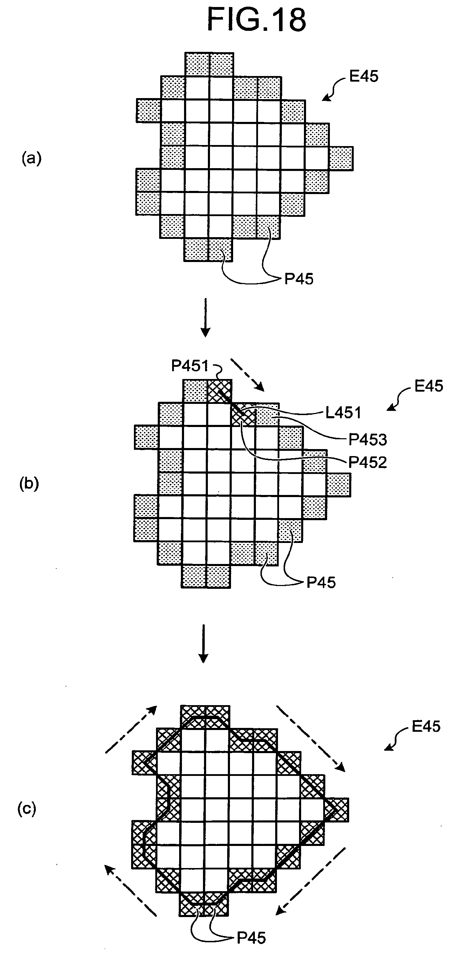

FIGS. 18(a) to 18(c) are explanatory diagrams for describing the principle of calculating the degree $\sigma$ of irregularity of the core boundary, illustrating the cell nucleus area E45. First, as illustrated in FIG. 18(a), contour pixels P45 of the cell nucleus area E45, indicated by hatched areas are extracted. Subsequently, the inclinations between adjacent contour pixels P45 are sequentially calculated. For example, as illustrated in FIG. 18(b), one contour pixel P451 is selected from the extracted contour pixels P45. First, a focus is made on the selected contour pixel P451 and a contour pixel P452 that is adjacent to the contour pixel P451 in a clockwise direction indicated by an arrow denoted by a one-dot chain line in FIG. 18(b), for example. Moreover, an inclination of a straight line L451 connecting these contour pixels P451 and P452 is calculated. Here, when the contour pixel P451 is a pixel A, the contour pixel P452 is a pixel B, the coordinate of the pixel A is $(x_A, y_A)$, and the coordinate of the pixel B is $(x_B, y_B)$, the inclination $a_{A,B}$ of the straight line L451 connecting the pixels A and B is expressed by the following equation (9).

$$a_{A,B} = \left| \frac{y_A - y_B}{x_A - x_B} \right| \qquad \cdots (9)$$

After that, a focus is moved such that the contour pixel P452 is set as the pixel A, and a contour pixel P453 adjacent to the contour pixel P452 in the clockwise direction is set as the pixel B, and the inclination between the pixels A and B is calculated. The same process is repeatedly performed so that the inclinations between all contour pixels P45 are calculated as illustrated in FIG. 18(c). Moreover, a variance of the obtained inclinations is calculated whereby the degree $\sigma$ of irregularity of the core boundary is obtained.

Moreover, the degree of circularity is calculated for each of the specified cell nucleus areas to obtain the degree C of circular deformation. Here, the degree of circularity amounts to the maximum when the shape of the cell nucleus area is a true circle. On the other hand, the degree of circularity has a smaller value as the contour shape becomes more complex. For example, the degree of circularity is calculated using the size of the cell nucleus area or the boundary length (the number of contour pixels).

Moreover, threshold processing is performed on the size S of the cell nucleus area, the degree $\sigma$ of irregularity of the core boundary, and the degree C of circular deformation calculated in this way, whereby the level of the nuclear atypicality is determined. Specifically, a threshold value $Th_S$ to be applied to the size S is set in advance, and it is determined whether the following expression (10) is satisfied. Moreover, a threshold value $Th_\sigma$ to be applied to the degree $\sigma$ of irregularity of the core boundary is set in advance, and it is determined whether the following expression (11) is satisfied. Moreover, a threshold value $Th_C$ to be applied to the degree C of circular deformation is set in advance, and it is determined whether the following expression (12) is satisfied. Moreover, the number corresponding to the corresponding expressions (10) to (12) among the respective values of the size S, the degree $\sigma$ of irregularity of the core boundary, and the degree C of circular deformation is determined as the level of the nuclear atypicality.

$$S > Th_S \tag{10}$$

$$\sigma > Th_\sigma \tag{11}$$

$$C \ll Th_S \tag{12}$$

Next, a fiber statistic amount will be described. Examples of the fiber statistic amount include a fiber density. First, closed regions made up of the fiber pixels are specified as individual fiber areas based on the component information on the fiber (elastic fiber) calculated as the feature amount. Specifically, fiber pixels are segmented in respective connecting components, and each of the segmented sets of pixels is specified as a fiber area. The connectivity is determined in a manner similar to the above-described method of specifying cell nucleus areas. In the case of fiber areas, although there is a possibility that multiple fiber areas cross each other, in this example, the fiber areas are specified by the same method as the case where the nucleus pixels are segmented in respective connecting components. That is, the respective pixels are labeled in the order of raster scanning. Thus, even if a part of a fiber area crosses or makes contact with another fiber area, since the fiber areas are labeled with different labels, they can be distinguished from each other.

Subsequently, a thinning process is performed so as to eliminate positional ambiguity of the fiber areas due to noise. By this thinning process, the skeletal line of the fiber area is acquired. In the subsequent processes, the fiber area can be treated as a linear region.

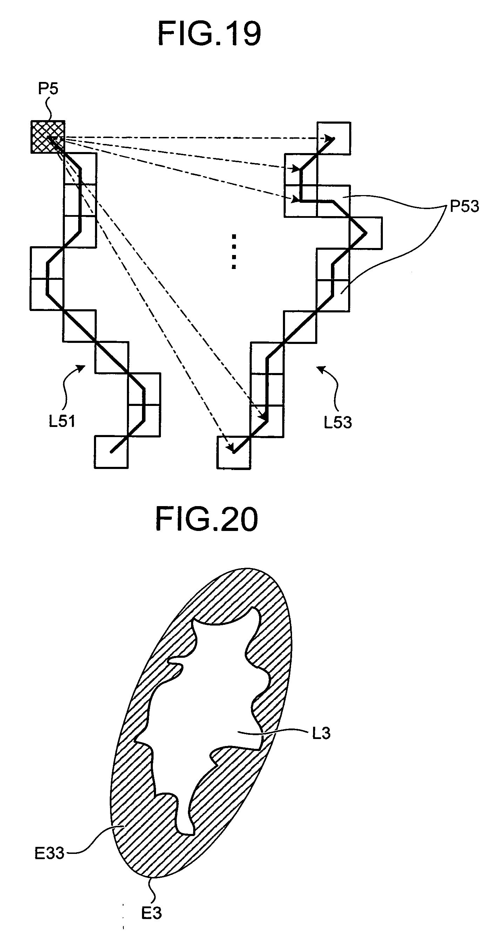

Subsequently, a fiber density is calculated based on a positional relation between the pixels constituting the skeletal line of the thinned fiber area and the pixels constituting the skeletal line of another fiber area. Specifically, an average distance to the other skeletal line with respect to all pixels constituting one skeletal line is calculated, whereby the fiber density is obtained. FIG. 19 is an explanatory diagram describing the principle of calculating the fiber density, in which the skeletal lines L51 and L53 of two fiber areas are illustrated. First, the distances between a pixel P5 constituting one skeletal line (skeletal line 1) L51 and all pixels P53 constituting the skeletal line (skeletal line 2) L53 are calculated. Moreover, the minimum value of the calculated distances is set as the distance $d_A$ between the pixel P5 and the skeletal line (skeletal line 2) L53. After that, similarly, the distances between all pixels constituting the skeletal line (skeletal line 1) L51 and the skeletal line (skeletal line 2) L53 are calculated, and the average value thereof is calculated as the average distance $AD_{1,2}$, that is, the fiber density.

When calculating the degree of shape irregularity of predetermined other tissues including fiber (for example, the gland structure (acinar structure) of the prostate), a normal tissue image may prepared in advance for each organ, and the shape feature amount of a normal tissue may be defined and stored in the storage unit 55. Moreover, depending on the type of an organ of the diagnosis target stained specimen set as the specimen attribute information, the normal tissue image and the shape feature amount of the corresponding organ may be read, and the degree of shape irregularity may be calculated by calculating the degree of similarity to the normal tissue.

Next, a blood vessel statistic amount will be described. Examples of the blood vessel statistic amount include a degree of shape irregularity. In calculation of the degree of shape irregularity, the contour calculated for the high-brightness region specified as the blood vessel area when specifying the blood vessel area and the ellipse obtained by approximating the high-brightness region to an ellipse are used.

FIG. 20 is an explanatory diagram for describing the principle of calculating the degree of shape irregularity, in which a high-brightness region (see FIG. 16) specified as the blood vessel area L3 and an ellipse E3 obtained by approximating the blood vessel area L3 which is the high-brightness region to an ellipse are illustrated. First, the area of a hatched region E33 in FIG. 20 surrounded by the contour line of the blood vessel area L3 and the ellipse E3 is calculated. Moreover, the percentage PS of the area of the region E33 to the area of the ellipse E3 is calculated, whereby the degree of shape irregularity is obtained. Here, when the area of the region E33 surrounded by the contour line of the blood vessel area L3 and the ellipse E3 is RS, and the area of the ellipse E3 is ES, the percentage PS is expressed by the following equation (13). The degree of shape irregularity which is the percentage PS indicates that the shape of the blood vessel is deformed more as the value decreases.

$$PS = \frac{RS}{ES} \tag{13}$$

After calculating the statistic amount for each diagnostic area in this way, as illustrated in FIG. 11, the cancer potential estimating unit 543 estimates the degree (cancer potential) of possibility that a portion photographed in each diagnostic area is a cancer based on the statistic amount of each diagnostic area calculated in step e5 (step e7). For example, the cancer potential estimating unit 543 compares the statistic amount of the diagnostic area with a general index (diagnosis index) that a pathologist determined during a diagnosis and past rare cases to thereby estimate the degree of possibility that the diagnostic area is a cancer. The diagnosis index is set in advance and stored in the storage unit 55. Specifically, the diagnosis index is the type of cancer that is likely to occur and a degeneration state of a cell nucleus, a fiber, and a blood vessel in the cancer. For example, the diagnosis index is set as a threshold value for the statistic amount or the value calculated from the statistic amount. Moreover, as for the past rare cases, the value of the statistic amount calculated for a stained specimen that has been diagnosed to be a rare case in the past, for example, is set. Moreover, the cancer potential is estimated in five levels, for example, of "Level 1: Possibility of cancer is very low," "Level 2: Only degenerated," "Level 3: Possibility of cancer is high," "Level 4: Certainly is cancer," and "Level 5: Suspected to be a rare case."

As an estimation order, for example, the statistic amount of the diagnostic area is compared with past rare cases to determine whether the diagnostic area is a rare case. When the diagnostic area is determined to be a rare case, the cancer potential is estimated as "Level 5." After that, as for diagnostic areas determined not to be a rare case, it is estimated whether the cancer potential is any one of "Level 1" to "Level 4" by referring to the diagnosis index.

Here, a method of estimating the cancer potential (the cancer potential of cervical squamous cell cancer) of "Level 1" to "Level 4" when "endocervix" is set as an organ type in the specimen attribute information, and "squamous epithelium" is set as a tissue will be described. As for the cervical squamous cell cancer, a diagnosis is made by observing a nuclear density and the degree of invasiveness to a blood vessel. Thus, threshold values for determining the nuclear density and the degree of invasiveness to the blood vessel are set as diagnosis indices, for example. First, the height of the nuclear density is determined in the following order based on the nucleus count and the inter-nucleus distance which are nucleus statistic amounts.

For example, the height of the nuclear density is determined by determining whether two conditions of the condition for the nucleus count and the condition for the inter-nucleus distance are satisfied.

First, the first condition for the nucleus count will be described. When the number of cell nucleus areas included in a virtual slide image is $Num_A$, and the number (nucleus count) of cell nucleus areas within a diagnostic area of ID = $\alpha$ is $Num_\alpha$, the percentage $R_N$ of the nucleus count within the diagnostic area to the total number of cell nuclei in the stained specimen is expressed by the following equation (14).

$$R_N = \frac{Num_\alpha}{Num_A} \qquad (14)$$

The condition for the nucleus count is whether the calculated $R_N$ satisfies the following expression (15).

$$R_N > Th_{R_N} \qquad (15)$$

That is, threshold processing is performed on the calculated percentage $R_N$. When the calculated percentage $R_N$ is greater than a threshold value $Th_{RN}$, it is determined that the condition for the nucleus count is satisfied.

Next, the second condition for the inter-nucleus distance will be described. When the area of the cell nucleus area in the diagnostic area of ID = $\alpha$ is Si (i = 1, 2, ..., $Num_\alpha$), the average area $\overline{S_\alpha}$ is expressed by the following equation (16). Here, "$S_\alpha^{-}$" represents that a symbol "-" representing the average value is attached over "$S_\alpha$."

$$\overline{S_\alpha} = \frac{\sum_{i=1}^{Num_\alpha} S_i}{Num_\alpha} \qquad \cdots (16)$$

Here, if the cell nucleus area is assumed to be circular, the radius $r_\alpha$ of the average area $S_\alpha^{-}$ is expressed by the following equation (17). Here, $\pi$ is a circular constant.

$$r_\alpha = \sqrt{\frac{\overline{S_\alpha}}{\pi}} \qquad\qquad \cdots (17)$$

Moreover, the condition for the inter-nucleus distance is determined by determining whether the following expression (18) is satisfied using the radius $r_\alpha$. Here, "d-" is the average value of the inter-nucleus distances calculated for the diagnostic areas of ID = $\alpha$, and "k" is a predetermined coefficient. Moreover, "d-" represents that a symbol "-" representing the average value is attached over "d."

$$\bar{d} < kr_\alpha \qquad\qquad (18)$$

A density level of "3" is obtained when both of the condition (the expression (15)) for the nucleus count and the condition (the expression (18)) for the inter-nucleus distance are satisfied, a density level of "1" is obtained when either one of the two conditions is satisfied, and a density level of "1" is obtained when neither of the two conditions is satisfied. The density level obtained herein indicates that the density is higher as the value increases.

Next, the degree of invasiveness to the blood vessel is determined based on the degree of shape irregularity which is the blood vessel statistic amount. As described above, the degree of shape irregularity indicates that the shape of the blood vessel is deformed more as the value decreases. Thus, in this example, threshold processing is performed on the value of the degree of shape irregularity, whereby the magnitude of the degree of invasiveness to the blood vessel is determined. For example, a threshold value $Th_{PS}$ for the degree of shape irregularity (that is, the percentage PS) is set in advance, and it is determined whether the condition of the following expression (19) is satisfied. Moreover, when the condition of the following expression (19) is satisfied, it is determined that the degree of invasiveness to the blood vessel is high, that is, the shape of the blood vessel is deformed.

$$PS < Th_{PS} \qquad\qquad (19)$$

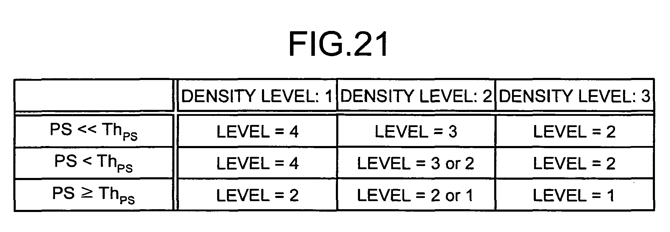

Moreover, the cancer potential is estimated based on combinations of the value of the density level and whether the value of the degree of shape irregularity is greater or smaller than the threshold value $Th_{PS}$. For example, a cancer potential estimation table in which the cancer potential is set in advance for each of the combinations is prepared and stored in the storage unit 55. Then, the cancer potential is estimated by referring to the cancer potential estimation table. FIG. 21 is a diagram illustrating an example of the cancer potential estimation table. In the example of FIG. 21, when the density level is "1" and the value of the degree of shape irregularity (the percentage PS) is very smaller than the threshold value $Th_{PS}$, the cancer potential is estimated as "Level 4." On the other hand, when the density level is "3" and the value of the degree of shape irregularity is the threshold value $Th_{PS}$ or more, the cancer potential is estimated as "Level 1."

The method of estimating the cancer potential is not limited to the above method. For example, a normal tissue image and a shape feature amount are set for each organ and stored in the storage unit 55. Moreover, the cancer potential may be estimated by calculating the degree of similarity to the normal tissue image and the shape feature amount. Moreover, the data used in estimating the cancer potential such as the diagnosis index, the normal tissue image and the shape feature amount described above may be stored in the storage unit 55, for example, as learning data.

After estimating the cancer potential for each diagnostic area in this way, as illustrated in FIG. 11, the cancer potential estimating unit 543 determines the presence of a diagnostic area in which the estimated cancer potential is "Level 4" Certainly is cancer." When a diagnostic area of "Level 4" is present (Yes in step e9), a grade is determined for the diagnostic area (step e11). When there is no diagnostic area of "Level 4," (No in step e9), the flow proceeds to step e13.

Here, the order of determining a grade in step e11 will be described. First, a deviation $\delta_{PS}$ to the condition of the expression (19) is calculated for each diagnostic area estimated to be "Level 4" by the following equation (20).

$$\delta_{PS} = Th_{PS} - PS \qquad\qquad (20)$$

After that, the values of the calculated deviation $\delta_{PS}$ are sorted to predetermined steps, and the grade is determined in accordance with the step to which the value of the deviation $\delta_{PS}$ is sorted. For example, first, deviation amounts of a predetermined number n of steps $step_i$ (i = 1, 2, ..., n) are determined based on the value of the deviation $\delta_{PS}$ for each diagnostic area. Here, i represents a step number. Moreover, the respective diagnostic areas are sorted to any of the steps based on the determined deviation amounts of the respective steps $step_i$. In the present embodiment, n = 5, for example. When the maximum value of the values of the deviation $\delta_{PS}$ of the respective diagnostic areas is deviation $\delta_{PS\_max}$, the deviation amount value (i) (i = 1, 2, ..., n) at the respective steps $step_i$ is expressed by the following expression (21).

$$(i-1)\times\left(\frac{\delta_{PS\_max}}{n}\right) < \text{value(i)} < i \times \left(\frac{\delta_{PS\_max}}{n}\right) \qquad \cdots (21)$$

Moreover, the respective diagnostic areas are sorted to any of the steps based on the determined deviation amounts value(i) of the respective steps $step_i$. The step sorted herein corresponds to the grade of the diagnostic area. The estimated cancer potential and the grade determined for the diagnostic area of which the cancer potential is "Level 4" are stored in the storage unit 55 as the information on the diagnostic area to which the diagnostic area ID is allocated.

Moreover, as illustrated in FIG. 11, in the subsequent step e13, the control unit 56 uses the positional information, the central position, the feature amount, the statistic amount, and the cancer potential for each diagnostic area, and the respective values of the grades determined for the diagnostic areas of which the cancer potential is "Level 4," stored in the storage unit 55 as the information on the diagnostic area as the result of the processes in steps e1 to e11 as the diagnostic area information, transmits the diagnostic area information to the stained specimen DB 4 together with the stained specimen ID, and sends a write request. In response to this request, the transmitted diagnostic area information is additionally registered in the stained specimen DB 4, and the stained specimen information on the diagnosis target stained specimen is updated. After that, the diagnostic area information creating process is finished, and the flow returns to step d3 of FIG. 10 and proceeds to a pathologist selecting process of step d5. FIG. 22 is a flowchart illustrating the flow of the pathologist selecting process.

As illustrated in FIG. 22, in the pathologist selecting process, first, a pathologist who is requested to make a diagnosis among the pathologists registered in the pathologist DB 6 is retrieved based on the specimen attribute information acquired in step d1 of FIG. 10 and the diagnostic area information created in the diagnostic area information creating process of FIG. 11 (step f1).

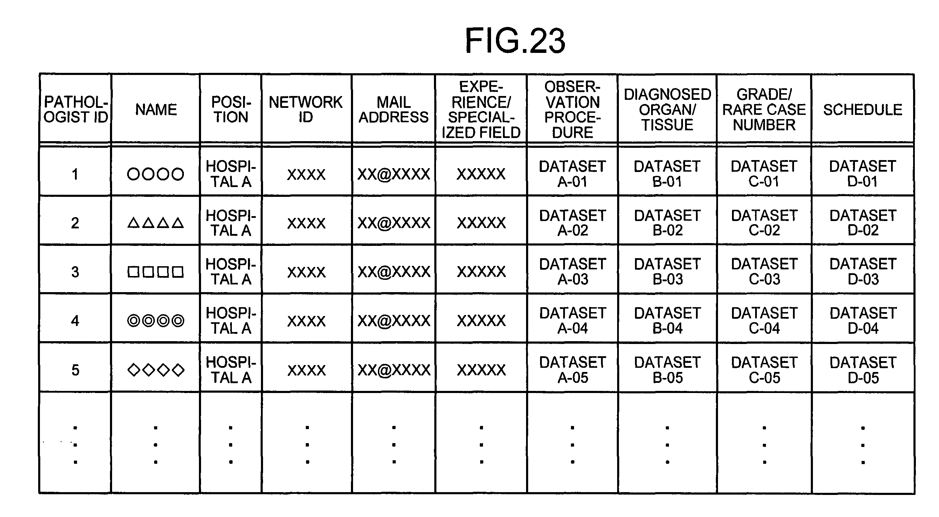

Here, the pathologist DB 6 will be described. In the pathologist DB 6, a position, contact address, an experience, and a specialized field of the pathologist, the type (diagnosed organ) of an organ that the pathologist has diagnosed in the past, a past case such as a case record for each grade and a case record of rare cases, and an observation procedure are listed and registered as pathologist information. FIG. 23 is a diagram illustrating a data configuration example of the pathologist DB 6. As illustrated in FIG. 23, a pathologist ID is stored in the pathologist DB 6 in correlation with the name of the corresponding pathologist, a position in which a medical facility of the place of work of the pathologist is set, a network ID of the pathology diagnosis device 7 of the pathologist, the mail address of the pathologist, the experience of the pathologist, the specialized field of the pathologist, the observation procedure (observation procedure information), the diagnosed organ/tissue and the grade/rare case number which is the past case (diagnosis record information) of the pathologist, and the schedule (schedule information) of the pathologist. The data of respective items constituting these sets of pathologist information are managed in correlation using a relational database, for example. The items constituting the pathologist information are not limited to those exemplified but can be set appropriately. Moreover, the data configuration of the pathologist DB 6 is not limited to this, but an optional data configuration can be used as long as the pathologist information corresponding to a value can be acquired by designating the value of each item.

Here, the observation procedure, the diagnosed organ/tissue, and the grade/rare case number, and the schedule are stored as datasets and are updated when the corresponding pathologist makes a diagnosis on a stained specimen, which will be described in detail.

The observation procedure (datasets A-01 to A-05, ...) stores the observation procedure of the corresponding pathologist. In the present embodiment, a dataset of a diagnosis image type is stored as the observation procedure, for example. Examples of the image type used during a diagnosis include a stained specimen RGB image, a dye amount image, a digitally stained image, and a pseudo-differential interference image. Although different types of images as exemplified above can be composed by modifying a virtual slide image, the type of image used for a diagnosis is different depending on a pathologist. That is, a pathologist may prefer observing and diagnosing dye amount images, and another pathologist may prefer observing and diagnosing digitally stained images. In the image type used during a diagnosis, the type of image that the corresponding pathologist used in the diagnosis is set. The dataset of the observation procedure is appropriately updated in response to a notification from the pathology diagnosis device 7 of the corresponding pathologist, for example.

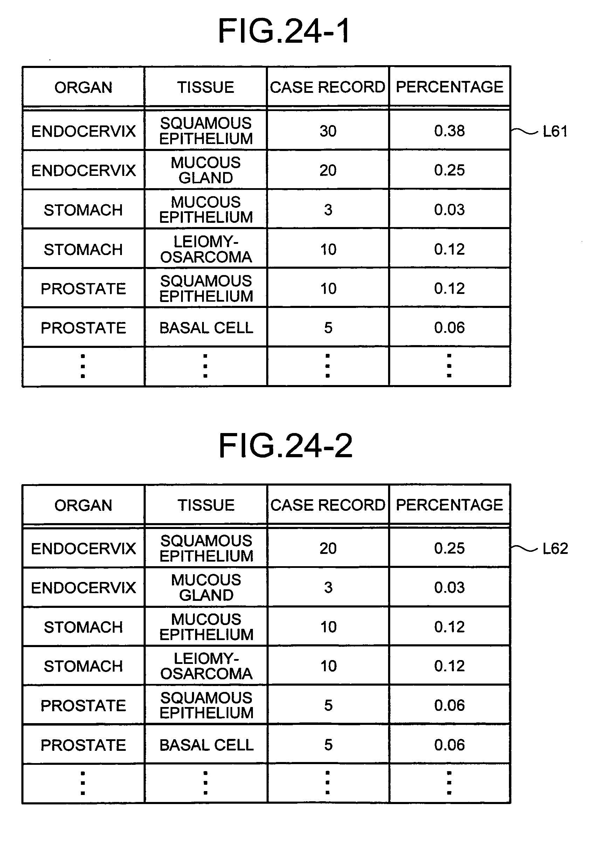

The diagnosed organ/tissue (datasets B-01 to B-05, ...) stores the organs and tissues that the corresponding pathologist has diagnosed in the past. FIG. 24-1 is a diagram illustrating an example of a dataset B-1 of the diagnosed organ/tissue of the pathologist information corresponding to the pathologist ID of "1" illustrated in FIG. 23. FIG. 24-2 is a diagram illustrating another example of a dataset B-2 of the diagnosed organ/tissue of the pathologist information corresponding to the pathologist ID of "2" illustrated in FIG. 23. As illustrated in FIGS. 24-1 and 24-2, a combination of the type of organ and the type of tissue is set in the dataset of the diagnosed organ/tissue in correlation with a case record (count) for the tissue of the organ and the percentage thereof. In the case count, the number of times of diagnosis made for the tissue of the organ in the past is set. In the percentage, the percentage of the case record (count) to the sum (total record count) of the case records for all combinations of the organs and the tissues is set. For example, in the record L61 for the squamous epithelium of the endocervix, "30" is set as the case record (count) for the squamous epithelium of the endocervix, and "0.38" is set as the percentage of the case record (count) of the squamous epithelium of the endocervix to the total record count.

Here, it is assumed that the pathologist corresponding to the pathologist information of FIG. 24-1 has made a diagnosis on a stained specimen in which the endocervix is set as an organ type, and the squamous epithelium is set as a target tissue type. In this case, the case record (count) in the record L61 for the squamous epithelium of the endocervix is added and updated, and the percentage is calculated and updated based on the updated case record (count). When there is no item (record) for the combination of a diagnosed endocervix and a diagnosed squamous epithelium, a record for a combination of an organ type and a target tissue type (in this example, a combination of the endocervix and the squamous epithelium) is added newly, and the case record (count) and the percentage thereof are set. As above, in the dataset (datasets B-01 to B-05, ...) of the diagnosed organ/tissue, when the corresponding pathologist makes a diagnosis on a stained specimen, the corresponding record is updated or a new record is added.

The grade/rare case number (datasets C-01 to C-05, ...) stores the grade and the rare case number of the diagnosed organ/tissue that the corresponding pathologist has made a diagnosis in the past. The grade is a value determined when the cancer potential is "Level 4" as described above, in which the case record (count) and the percentage are stored for each grade. That is, when the corresponding pathologist makes a diagnosis on a stained specimen of which the cancer potential is "Level 4," the case record of that grade is added, and the percentage is updated. The rare case number stores the case record (count) for rare cases. Specifically, when the corresponding pathologist makes a diagnosis on a stained specimen of which the cancer potential is "Level 5," the case record is added.

The schedule (datasets D-01 to D-05, ...) stores a predetermined period (for example, one month) of schedules of the corresponding pathologist. The dataset of the schedule is updated to the latest information at an appropriate time.

In step f1 of FIG. 22, the pathologist retrieving unit 561 retrieves a pathologist who is suitable for making a diagnosis on a diagnostic area from the pathologist DB 6 having the above-mentioned configuration. Here, a method of retrieving a pathologist will be described by focusing on one diagnostic area (for example, a diagnostic area ID = $ID_\beta$). FIG. 25 is a diagram illustrating an example of the specimen attribute information of a diagnosis target stained specimen and the diagnostic area information of a target diagnostic area. In the diagnosis target stained specimen illustrated in FIG. 25, the organ type is endocervix, the target tissue type is squamous epithelium, and the staining method is H&E staining. Moreover, the cancer potential estimated for the target diagnostic area of the diagnostic area ID = $1_\beta$ is "Level 4," and the grade is "II." Moreover, "1 (Urgent)" is set as the urgency level.

In the present embodiment, the pathologist is retrieved based on an organ type, a target tissue type, and a cancer potential (including a grade when the grade is set), for example, among the specimen attribute information of the diagnosis target stained specimen and the diagnostic area information of the target diagnostic area. The combination of items used for the retrieval is not limited to this, but the pathologist may be retrieved appropriately using other items or a combination of other items.

First, a pathologist in which a high percentage is set for the organ type and the target tissue type of the diagnosis target stained specimen is selected by referring to the dataset (the datasets B-01 to B-05, ... of FIG. 23) of the diagnosed organ/tissue of all sets of the pathologist information registered in the pathologist DB 6. For example, when the organ type set in the diagnostic area information is endocervix, and the target tissue type is squamous epithelium (see FIG. 25), the record L61 is referred to as for the dataset B-1 of the pathologist information illustrated in FIG. 24-1. On the other hand, the record L62 is referred to as for the dataset B-2 of the pathologist information illustrated in FIG. 24-2. In these records L61 and L62, since the value of the percentage of the record L61 is larger, the pathologist (pathologist ID = 1) of the dataset B-1 is selected. However, since a large number of sets of pathologist information are registered in the pathologist DB 6, in this example, the top N pathologists in which a high percentage is set to the organ type and the target tissue type of the diagnosis target stained specimen within the respective sets of pathologist information are selected. In the following description, N is set to 5 (top 5 pathologists) when the cancer potential is other than "Level 4." When the cancer potential is "Level 4," N is set to a value of 5 or more, for example, 10 (top 10 pathologists). The reason why a large N value is set when the cancer potential is "Level 4" is to further narrow the pathologists based on the grade as will be described later. The value of N is not particularly limited, and a value of 2 or more can be appropriately set. In this example, although the top N pathologists of which the percentage value are on the higher rank have been selected, a threshold value may be set in advance, and pathologists of which the percentage values are greater than the threshold value may be selected. Moreover, in this example, although the pathologist has been selected based on the value of the percentage set in the dataset of the diagnosed organ/tissue, the pathologist may be selected based on the value of the diagnosis record (count).

Subsequently, when the cancer potential is "Level 4," the next process is performed. That is, the top M pathologists in which a high diagnosis record (count) or a high percentage is set to the grade of the diagnostic area information are selected by referring to the datasets (the datasets C-01 to C-05, ... of FIG. 23) of the grade/rare case number of the selected pathologist information. For example, the maximum value of M is set to 5, and M pathologists at most are selected. The value of M is not particularly limited, and a value of 2 or more can be appropriately set.

After that, as illustrated in FIG. 22, the pathologist retrieving unit 561 selects a request candidate pathologist by referring to the datasets (the datasets D-01 to D-05, ... of FIG. 23) of the schedule of the pathologists narrowed in this way (step f3). For example, a predetermined number (for example, in the present embodiment, three pathologists, or at least one pathologist if the number of corresponding pathologists is less than 3) of pathologists who have a vacant time in the schedule in a predetermined diagnosis period (for example, 3 days) after the retrieved date are selected from the narrowed pathologists as request candidate pathologists. In this example, although three pathologists at most have been selected, the number of selected pathologists can be set appropriately as long as at least one pathologist is selected.

When there is no corresponding pathologist, the urgency level (see FIG. 25) set in the specimen attribute information is determined. When the value of the urgency level is a predetermined threshold value or less (for example, 2 or less, that is "1 (Urgent)" or "2 (Pressing)"), and a diagnosis is pressing, the process is branched depending on whether the cancer potential is "Level 4" or not. That is, when the cancer potential is other than "Level 4," pathologists who are on the lower rank than the top N-th rank are newly selected. In this example, pathologists on the ranks lower than the top 6th rank are sequentially selected, and it is determined whether the pathologist has a vacant time by referring to the dataset of the schedule. Moreover, the pathologists who have a vacant time are selected as the request candidate pathologists. When the cancer potential is "Level 4," pathologists who are on the lower rank than the top M-th rank are newly selected, and in a manner similar to the case where the cancer potential is other than "Level 4," pathologists who have a vacant time in the schedule are selected as the request candidate pathologists. On the other hand, when the value of the urgency level is greater than a predetermined threshold value (for example, "3 (Normal)"), and an urgent diagnosis is not required, a vacant time in the schedule is determined while extending the diagnosis period by a predetermined period (for example, 2 days) up to 1 month, and the request candidate pathologists are selected. The process of step f3 ends when a predetermined number of request candidate pathologists have been selected.

Although a method of retrieving the pathologist by focusing on one diagnostic area has been described, when multiple diagnostic areas are extracted, a diagnostic area having the highest level of cancer potential is specified by referring to the cancer potential set to the respective diagnostic areas. Moreover, the above-mentioned process is performed using the specified diagnostic area having the highest level of cancer potential, and the request candidate pathologists are selected.

The method of selecting the request candidate pathologists is not limited to the above method. For example, when the cancer potential is a specific level, the request candidate pathologists may be selected based on the experience. For example, as for "Cancer potential: Level 1," a pathologist who has a small number of years of diagnosis experience may be selected to be requested to make a diagnosis based on the experience.

Subsequently, the diagnosis request acceptability determining unit 562 sends a request for reply to diagnosis request to the pathology diagnosis device 7 of the request candidate pathologist selected in step f3 (step f5). Specifically, the diagnosis request acceptability determining unit 562 sends the request for reply to diagnosis request to the pathology diagnosis device 7 in accordance with the pathologist ID, the name, the position, the network ID, and the mail address set in the pathologist information of the request candidate pathologist. When multiple request candidate pathologists are selected, the request for reply to diagnosis request is sent to the respective pathology diagnosis devices 7 of the request candidate pathologists.

In response to the request, the pathology diagnosis device 7 having received the request for reply to diagnosis request displays the request for reply to diagnosis request to prompt selection of the diagnosis acceptability. A waiting state is continued until the selection of the diagnosis acceptability is input (No in step g1). When the selection of the diagnosis acceptability is input (Yes in step g1), acceptability information in which selection content ("Acceptable" or "Non-acceptable (Reject)") is set is sent to the information distributing device 5 (step g3).

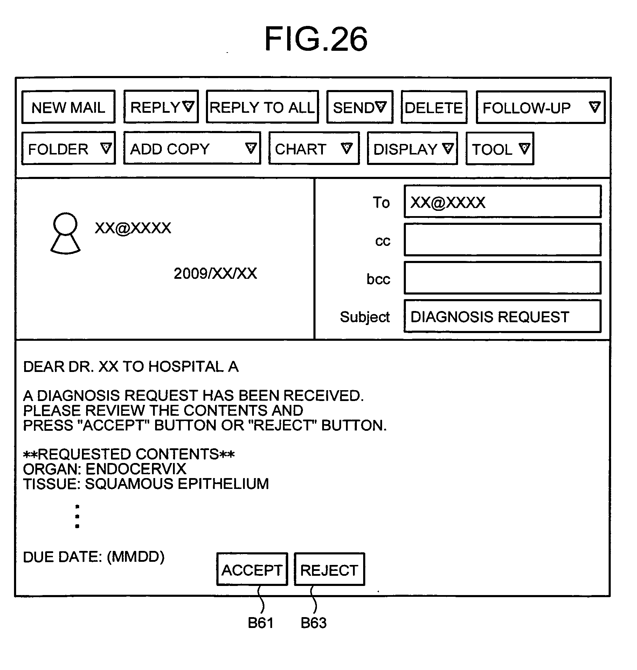

FIG. 26 is a diagram illustrating an example of the request for reply to diagnosis request displayed on the screen of the pathology diagnosis device 7. As illustrated in FIG. 26, the request for reply to diagnosis request is sent through a mail, for example, and is presented to a pathologist by being displayed on the screen as a message prompting the selection of "Accept" button B61 or "Reject" button B63. The pathologist checks the request content and a reply deadline and replies to the request for reply to diagnosis request by clicking on the "Accept" button B61 when accepting the diagnosis or clicking on the "Reject" button B63 when rejecting the diagnosis. Here, when the "Accept" button B61 is clicked, the acceptability information in which "Acceptable" is set is transmitted to the information distributing device 5. When the "Reject" button B63 is clicked, the acceptability information in which "Non-acceptable" is set is transmitted to the information distributing device 5.

On the other hand, when the information distributing device 5 receives the acceptability information (Yes in step f7), the diagnosis request acceptability determining unit 562 determines the number of pathologists who have accepted the request based on the acceptability information. That is, when "Acceptable" is set in the received acceptability information, the pathologist is determined as the request pathologist, and the number of determined request pathologists is added. Moreover, the diagnosis request acceptability determining unit 562 receives the acceptability information by returning to step f7 until the number of pathologists (the number of determined request pathologists) who have accepted the request reaches an upper limit count (for example, 3) (No in step f9). In this way, the request pathologist is determined from five request candidate pathologists in the order of arrival. When the number of request candidate pathologists selected in step f3 is less than the upper limit count (3), all pathologists who have accepted the request may be determined as the request pathologists. In this example, although the upper limit count is set to 3, the upper limit count may be appropriately set as long as it is 1 or more.

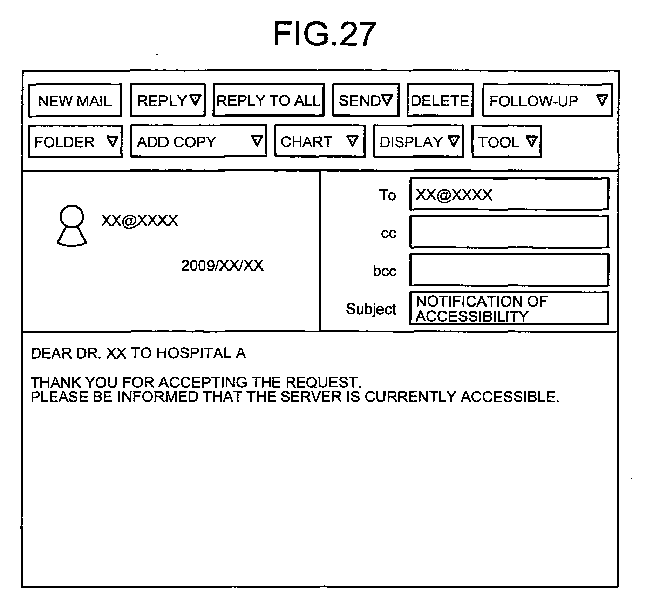

More specifically, when "Acceptable" is set in the received acceptability information, and the pathologist of the pathology diagnosis device 7 having sent the acceptability information is determined as the request pathologist, the diagnosis request acceptability determining unit 562 allocates an access right to the network ID of the pathology diagnosis device 7 to realize a state where the stained specimen information of the diagnosis target stained specimen can be accessed. After that, a notification is sent to inform that the pathology diagnosis device 7 can be accessed. FIG. 27 is a diagram illustrating an example of a notification of accessibility displayed on the screen of the pathology diagnosis device 7. As illustrated in FIG. 27, this notification is sent through a mail and presented to the pathologist in a manner similar to the request for reply to diagnosis request.

When it is determined in step f9 of FIG. 22 that the upper limit count has reached (Yes in step f9), the diagnosis request acceptability determining unit 562 determines the request pathologists (step f11).

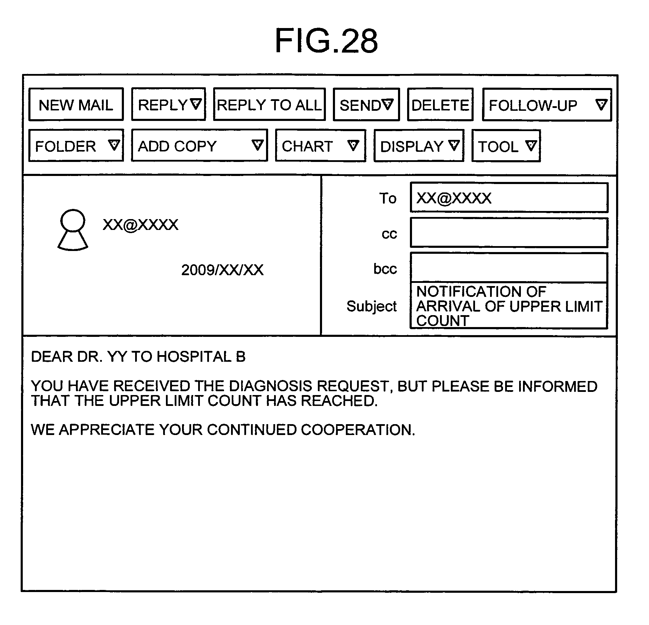

More specifically, in this case, a notification of arrival of the upper limit count is sent to the pathology diagnosis device 7 of a request candidate pathologist from which the acceptability information has not been received among the request candidate pathologists to which the request for reply to diagnosis request has been sent. FIG. 28 is a diagram illustrating an example of a notification of arrival of the upper limit count displayed on the screen of the pathology diagnosis device 7. As illustrated in FIG. 28, this notification is sent via a mail and presented to the pathologist in a manner similar to the request for reply to diagnosis request.

Subsequently, in step f13 of FIG. 22, the image modification processing unit 545 of the providing information creating unit 544 processes (modifies) the virtual slide image in accordance with an observation procedure to create providing information by referring to the dataset of the observation procedure of the request pathologist determined in step f11. The process of step f13 is performed for each request pathologist, and the providing information suitable for the respective request pathologist is created.

Specifically, when the stained specimen RGB image is set as the diagnosis image type, the image data of the diagnostic area in the stained specimen RGB image is cut based on the positional information, and the cut image data is modified by image processing to thereby create the providing information.

Here, an RGB image (normalized RGB image) in which the dye amount is normalized may be generated using the color information correction coefficients calculated as the feature amount of the diagnostic area. Specifically, first, the dye amounts of the dyes H and E of the respective pixels constituting the diagnostic area are adjusted in accordance with the color information correction coefficients. Specifically, the color information correction coefficients are set to $\alpha_H$ and $\alpha_E$, and the dye amounts $d_H$ and $d_E$ are multiplied by the color information correction coefficients $\alpha_H$ and $\alpha_E$, whereby the dye amounts are adjusted. The calculation formulas of the adjusted dye amounts $d_H^*$ and $d_E^*$ are expressed by the following equations (22) and (23).

$$d^\star{}_H = \alpha_H d_H \qquad\qquad (22)$$

$$d^\star{}_E = \alpha_E d_E \qquad\qquad (23)$$

Moreover, the dye amounts $d_H^*$ and $d_E^*$ adjusted in this way are substituted into the equation (4), and the obtained value is converted into a spectral transmittance in accordance with the following equation (24). In this way, the spectral transmittances at the respective pixel positions are obtained from the adjusted dye amounts $d_H^*$ and $d_E^*$. After calculating the spectral transmittances for the respective pixels in the diagnostic area in the above-described method, the RGB values $G_{RGB}(x)$ of the respective pixels are calculated in accordance with the equations (2) and (3), and the normalized RGB images are composed based on the adjusted dye amounts $d_H^*$ and $d_E^*$. Moreover, the above-described process is performed for each diagnostic area, whereby the normalized RGB images of the respective diagnostic areas are created as the providing information.

$$t\left(x, \lambda\right) = e^{-\left(k_H\left(\lambda\right)d^*_H\left(x\right)+k_E\left(\lambda\right)d^*_E\left(x\right)\right)} \qquad \cdot \cdot \cdot (24)$$

By adjusting the dye amounts using the color information correction coefficients $\alpha_H$ and $\alpha_E$ in this way, it is possible to correct the RGB image into an image having colors equivalent to the stained specimen stained at a desired density. Thus, a value preferred by the pathologist may be set as the values of the color information correction coefficients $\alpha_H$ and $\alpha_E$. For example, the values of the color information correction coefficients $\alpha_H$ and $\alpha_E$ may be set as the observation procedure of the pathologist depending on whether the pathologist prefers observing the stained specimen stained at a standard density, a high density, or a low density. Moreover, the dye amounts of the respective pixels constituting the diagnostic area may be adjusted in a manner as described above based on the values of the color information correction coefficients $\alpha_H$ and $\alpha_E$ set as the observation procedure of the request pathologist, and the RGB images of the diagnostic area may be composed based on the adjusted dye amounts to thereby create the providing information.

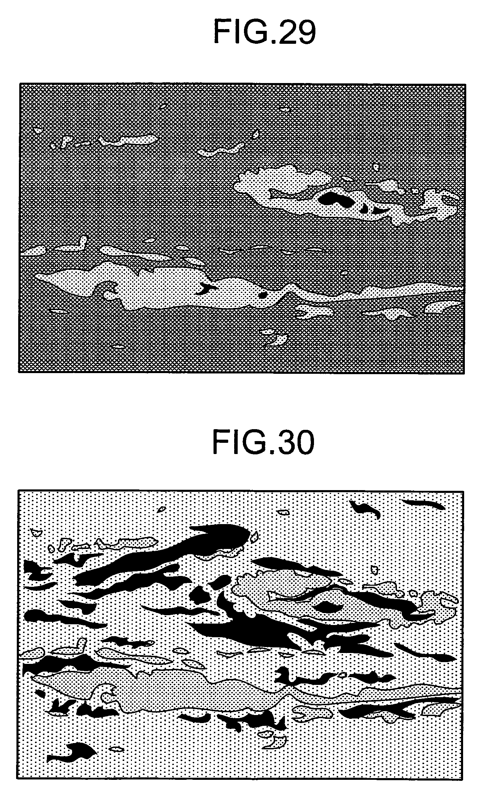

On the other hand, when the dye amount image is set as the diagnosis image type, the dye amount image of the diagnostic area is generated. In the present embodiment, since the H&E stained specimen is used as the diagnosis target, the dye amounts of the dye H at the respective pixel positions of the diagnostic area are read based on the positional information, and the H-dye amount image expressed by the density is generated. Similarly, the dye amounts of the dye E at the respective pixel positions of the diagnostic area are read, and the E-dye amount image expressed by the density is generated. FIG. 29 is a diagram illustrating an example of the H-dye amount image of the diagnostic area. Moreover, FIG. 30 is a diagram illustrating an example of the E-dye amount image of the diagnostic area.

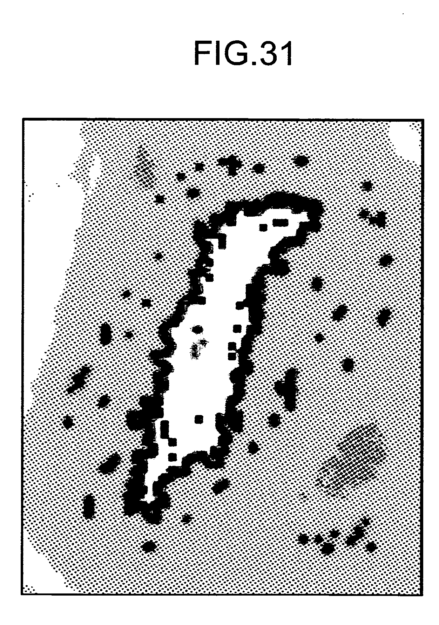

Moreover, when the digitally stained image is set as the diagnosis image type, the digitally stained image of the diagnostic area is generated. Here, the digitally stained image is an image in which a desired component such as a cell nucleus, a fiber, or a blood vessel is highlighted as if it were subjected to special staining. Specifically, first, the image data of the diagnostic area in the stained specimen RGB image is cut based on the positional information. Subsequently, the pixels of the cell nucleus, the fiber, and the blood vessel are extracted from the image data of the diagnostic area based on the identification pixel conditions of the respective components by referring to the component information created as the feature amount of the diagnostic area. Moreover, the display colors of the pixels of the cell nucleus, the fiber, and the blood vessel in the diagnostic area of the stained specimen RGB image are substituted with a predetermined display color, whereby a digitally stained image is generated so that the components in the diagnostic area are highlighted so as to be distinguished from the other components. In this case, it is not necessary to change the display colors of all of the pixels of the cell nucleus, the fiber, and the blood vessel, but the display color of the pixels of only a predetermined component may be changed. Which component will be highlighted may be determined based on the user's operation received via the input unit 51, for example. Moreover, when multiple components are highlighted, the respective pixels may be substituted with different display colors. FIG. 31 is a diagram illustrating an example of the digitally stained image in which an elastic fiber is highlighted.

Moreover, when the pseudo-differential interference image is set as the diagnosis image type, the pseudo-differential interference image of the diagnostic area is generated. The pseudo-differential interference image is generated by combining the virtual slide images of which the focus positions are different. Specifically, for example, the front focus position and the back focus position other than the in-focus position measured in step c13 of FIG. 6 are used as the focus positions $F_\alpha$ and $F_\beta$. Moreover, by referring to the spectral transmittances calculated for the respective pixel positions of the virtual slide image at the front focus position and the back focus position, a logical product between the same pixels is calculated by allocating a logical value of "1" to pixels of which the spectral transmittance at a predetermined wavelength $\lambda$ is the threshold value or higher and a logical value of "0" to pixels of which the spectral transmittance is lower than the threshold value. Moreover, the pixel value of pixels satisfying the logical product is set to "255" and the pixel value of pixels not satisfying the logical product is set to "0," whereby the pseudo-differential interference image is generated.

In this example, the image data of the diagnostic area is cut, and the cut image data is modified by image processing to thereby create the providing information. In contrast, the entire area of the stained specimen image may be modified to create the providing information.

After creating the providing information in this way, the providing information creating unit 544 transmits the created providing information to the stained specimen DB 4 together with the stained specimen ID and sends a write request (step f15). In response to this request, the transmitted providing information is additionally registered in the stained specimen DB 4, and the stained specimen information on the diagnosis target stained specimen is updated. After that, the pathologist selecting process is finished, and the flow returns to step d5 of FIG. 10 and proceeds to step d7.

In step d7, the providing information distribution processing unit 563 uses the stained specimen information including the specimen attribute information and the image data of the stained specimen image acquired in step d1, the diagnostic area information created by the diagnostic area information creating process of step d3, and the providing information created in step f13 of FIG. 22 as the diagnostic information and distributes the diagnostic information to the pathology diagnosis device 7 of the request pathologist determined in step f11 of FIG. 22. The process of step d7 may not be performed right after the pathologist selecting process of step d5, but may be performed at an appropriate time, for example, when the pathology diagnosis device 7 of the request pathologist accesses the information distributing device 5 and sends a diagnosis start request to the information distributing device 5.

In response to this, the pathology diagnosis device 7 displays the specimen attribute information, the image data of the stained specimen image, the diagnostic area information, and the providing information, which is the received diagnostic information, on the screen (step h1). The pathologist of the pathology diagnosis device 7 makes an observation and a diagnosis while viewing the diagnostic information such as the providing information displayed on the screen and inputs a diagnosis result by operating an input device. Moreover, the pathology diagnosis device 7 creates diagnosis report information in accordance with an operation input (step h3).

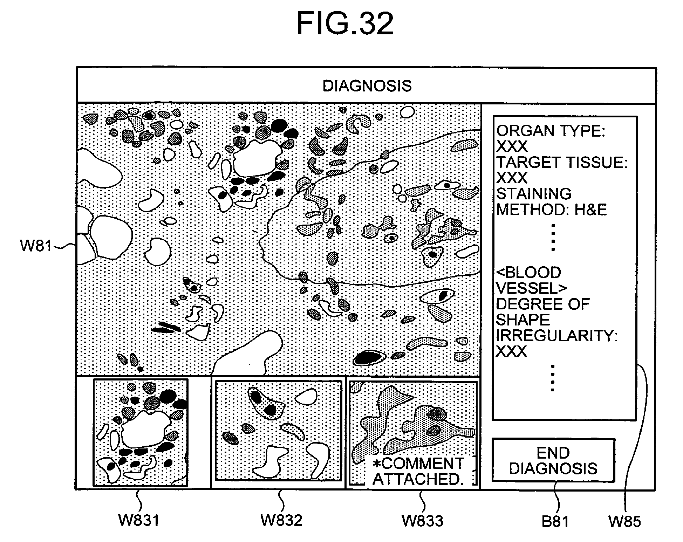

For example, the pathology diagnosis device 7 displays a diagnosis screen, in which the specimen attribute information, the image data of the stained specimen image, the diagnostic area information, and the providing information are included, based on the received diagnostic information. FIG. 32 is a diagram illustrating an example of the diagnosis screen. As illustrated in FIG. 32, the diagnosis screen includes an entire image display portion W81, diagnostic area display portions W831, W832, and W833, and an auxiliary information display portion W85. Moreover, the diagnosis screen includes an End Diagnosis button B81.

In the entire image display portion W81, a stained specimen RGB image generated based on a virtual slide image obtained by combining sectioned specimen area images which are high-resolution images is displayed. The stained specimen RGB image displayed in the entire image display portion W81 can be partially enlarged or reduced by selecting an enlarge menu or a reduce menu (not illustrated). The request pathologist of the pathology diagnosis device 7 can observe an entire area or partial areas of the diagnosis target stained specimen with high resolution in the entire image display portion W81 by the similar method as a method of actually observing the diagnosis target stained specimen using a high-magnification objective lens and the virtual slide microscope 2.

In the diagnostic area display portions W831, W832, and W833, the providing information of each diagnostic area is displayed. In the present embodiment, the providing information is obtained by modifying the image data of each diagnostic area by image processing as described above. For example, when the diagnosis image type is set to "stained specimen RGB image" as the observation procedure of the request pathologist of the pathology diagnosis device 7, the image data of the diagnostic area cut from the stained specimen RGB image is distributed as the providing information, and the respective sets of the providing information of the respective diagnostic areas are displayed in the diagnostic area display portions W831, W832, and W833, respectively. The image data of the diagnostic areas displayed in the diagnostic area display portions W831, W832, and W833 are appropriately displayed in an enlarged scale in the entire image display portion W81 in accordance with the user's operation.

Moreover, in the diagnostic area extraction screen of FIG. 12, for example, when a user (a requesting pathologist) has written a comment on the diagnostic area, "Comment Attached" is displayed as illustrated in the diagnostic area display portion W833, for example. When the display of "Comment Attached" is clicked, the detailed content thereof is displayed. The request pathologist can make a diagnosis while viewing the content of the comment (the opinions, queries, and questions of the requesting pathologist).

In the auxiliary information display portion W85, the content of the specimen attribute information and the diagnostic area information received together with the providing information are displayed as a list. The request pathologist can refer to the content of the specimen attribute information and the diagnostic area information in the auxiliary information display portion W85. Here, for example, a pathologist may prefer checking the shape information of a cell nucleus, a fiber, or a blood vessel, and another pathologist may prefer checking other feature amounts or statistic amounts. Thus, since the request pathologist can make a diagnosis while appropriately referring to necessary values among the feature amount or the statistic amount of the diagnostic area, it is possible to quickly make a diagnosis.

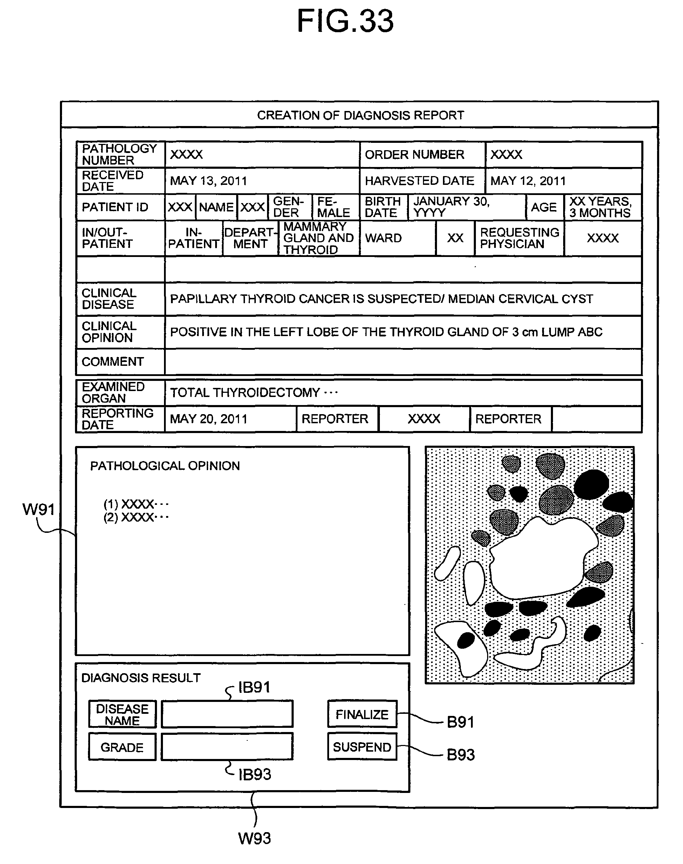

In the diagnosis screen, when the diagnostic area display portion W831 is double-clicked, for example, a report creation screen is displayed, in which a pathologist inputs opinions or diagnosis results on the corresponding diagnostic area. FIG. 33 is a diagram illustrating an example of the report creation screen. As illustrated in FIG. 33, in the report creation screen, the specimen attribute information such as patient information, or necessary items within the pathologist information of a request pathologist who made the diagnosis, and the image data (providing information) of the double-clicked diagnostic area are displayed. Moreover, the report creation screen includes an opinion input portion W91 and a diagnosis result input portion W93.

Here, the diagnosis result input portion W93 includes an input box IB91 for inputting a disease name, an input box IB93 for inputting a grade, and a Finalize button B91 and a Suspend button B93 for selecting whether the diagnosis will be finalized or suspended. The request pathologist clicks the Finalize button B91 when the request pathologist has confidence in a disease name, a grade, and the like. On the other hand, the request pathologist clicks the Suspend button B93 when the request pathologist does not have confidence.

In the report creation screen, the request pathologist writes an opinion on the diagnostic area in the opinion input portion W91. Moreover, when a comment on the corresponding diagnostic area has been written by the requesting pathologist, and the content thereof is a query or a question to the request pathologist, a response to the query or the question is appropriately written in the opinion input portion W91. Moreover, the request pathologist inputs a disease name and a grade in the input boxes IB91 and IB93 of the diagnosis result input portion W93, and clicks the Finalize button B91 or the Suspend button B93 to thereby end the diagnosis on the corresponding diagnostic area.

When the Finalize button B91 or the Suspend button B93 is clicked, the display returns to the diagnosis screen of FIG. 32. The request pathologist performs the same operations to input an opinion and a diagnosis result for each diagnostic area. When the pathologist finishes the diagnosis for all diagnostic areas, the pathologist clicks the End Diagnosis button B81 to end the diagnosis. When the End Diagnosis button B81 is clicked, the content of the opinion and the diagnosis result for each diagnostic area is created as the diagnosis report information. The pathologist can modify the written opinions and the input diagnosis results until the pathologist clicks the End Diagnosis button B81. That is, when the diagnostic area display portion W831 is clicked, the report creation screen of the corresponding diagnostic area is displayed again so that the pathologist can modify, overwrite, or delete the written opinion or the input diagnosis result.

After creating the diagnosis report information in this way, as illustrated in FIG. 10, the pathology diagnosis device 7 transmits the diagnosis report information to the information integrating device 8 together with the stained specimen ID (step h5).

Moreover, the pathology diagnosis device 7 uses the specimen attribute information and the diagnosis report information as the diagnosis content information, transmits the diagnosis content information to the pathologist DB 6 together with the pathologist ID of the pathologist of the pathology diagnosis device 7, and sends a write request (step h7). In response to this, in the pathologist DB 6, the dataset of the diagnosed organ/tissue of the corresponding pathologist information is updated in accordance with the organ type and the target tissue type of the specimen attribute information. Moreover, when the cancer potential is "Level 4," and the grade is determined, or when the cancer potential is "Level 5," and the diagnostic area is a rare case, the dataset of the grade/rare case number is updated.

On the other hand, the information integrating device 8 acquires the stained specimen information of the diagnosis target stained specimen from the stained specimen DB 4 based on the stained specimen ID received together with the diagnosis report information (step i1). Moreover, the information integrating device 8 integrates the diagnosis report information received from the pathology diagnosis device 7 and the stained specimen information acquired in step i1 to create final diagnosis result information (step i3). Here, when multiple request pathologists are determined in step f11 of FIG. 22, and the diagnostic information is distributed to the pathology diagnosis devices 7 of the multiple request pathologists in step d7 of FIG. 10, the information integrating device 8 receives the diagnosis report information from the pathology diagnosis devices 7 of the respective request pathologists. In this case, the information integrating device 8 integrates the respective sets of the received diagnosis report information from the pathology diagnosis devices 7 of the respective request pathologists to create the final diagnosis result information.

Moreover, the information integrating device 8 transmits the created final diagnosis result information to the stained specimen DB 4 together with the stained specimen ID and sends a write request (step i5). In response to this, the transmitted final diagnosis result information is additionally registered in the stained specimen DB 4, and the stained specimen information on the diagnosis target stained specimen is updated.

As described above, according to the diagnostic information distribution device of the present embodiment, a request pathologist who is requested to make a diagnosis is selected from pathologists who operate multiple pathology diagnosis devices, the image data of at least the diagnostic area extracted from the specimen image is subjected to image processing corresponding to a predetermined observation procedure of the request pathologist, whereby the providing information can be created. Moreover, it is possible to distribute the diagnostic information including the created providing information to the pathology diagnosis device of the request pathologist. Thus, since the request pathologist of the pathology diagnosis device having received the diagnostic information can make a diagnosis by a familiar observation procedure, the pathologist can quickly make a diagnosis.

Moreover, according to the pathology diagnosis system 1 of the present embodiment, it is possible to extract a diagnostic area in a stained specimen image, which is to be sent to another pathologist to obtain an opinion thereon. Moreover, it is possible to calculate a feature amount and a statistic amount of the diagnostic area and estimate a cancer potential to determine a grade.

Further, according to the pathology diagnosis system 1 of the present embodiment, it is possible to retrieve pathologists based on the specimen attribute information of the diagnosis target stained specimen, the diagnostic area information such as the cancer potential estimated based on the statistic amount of the diagnostic area, for example, and the schedule of the pathologist and to determine a request pathologist.

Here, in order to select a pathologist to whom a user (attending pathologist) sends a request for an opinion, a method of displaying the specialized field and the experience of pathologists who are consultable at that time on a screen and selecting one from the pathologists displayed on the screen may be used. However, it is difficult to determine whether the pathologist has a diagnosis record or knowledge on a case, on which an opinion is sought, just by the specialized field and the experience. Thus, in order to actually select a pathologist who is sought for an opinion, it is necessary to consider which case the pathologist has a diagnosis record, whether the pathologist has diagnosed a case equivalent to the degree of pathological malignancy of a diagnosis target, or whether the pathologist has knowledge on a rare case. Moreover, it is necessary to select a request pathologist by considering whether the pathologist has a vacant time for making a diagnosis. When the above-mentioned information is presented for the respective pathologists, the attending pathologist has to select a desired pathologist from a large amount of information, which makes the operation complicated and consumes a lot of time. In contrast, according to the present embodiment, the user (requesting pathologist) can automatically select a pathologist who is optimal for providing an opinion on the diagnosis as a request pathologist just by extracting the diagnostic area. Thus, it is possible to prevent complicated operations necessary for retrieving the request pathologist.

Moreover, according to the pathology diagnosis system 1 of the present embodiment, it is possible to create the providing information by processing the image data of at least the diagnostic area in accordance with the observation procedure of the determined request pathologist. Moreover, it is possible to distribute the diagnostic information including the providing information to the pathology diagnosis device of the request pathologist. Thus, after the image data of the diagnostic area can be modified by image processing into an image of the type which the request pathologist is familiar with when making a diagnosis, the modified image can be distributed to the pathology diagnosis device 7 of the request pathologist.

Therefore, according to the pathology diagnosis system 1 of the present embodiment, since the request pathologist can diagnose a similar case which belongs to the specialized field (strong field) of the request pathologist, and on which the request pathologist has a diagnosis record, in a normal diagnosis environment, it is possible to make a diagnosis efficiently. Accordingly, since a consultation such as a second opinion can be quickly made, it is possible to shorten the time up to the end of diagnosis and start treatment quickly.

Moreover, according to the present embodiment, it is possible to calculate and estimate the feature amount, the statistic amount, and the cancer potential with respect to at least the diagnostic area, on which the requesting pathologist wants to seek an opinion to include the obtained data in the diagnostic information, and to distribute the diagnostic information to the pathology diagnosis device of the request pathologist. Thus, the request pathologist can perform operations efficiently while appropriately referring to the information and to quickly make a diagnosis.

In the above embodiment, although the stained specimen RGB image, the dye amount image, the digitally stained image, or the pseudo-differential interference image has been created as the providing information based on the diagnosis image type set as the observation procedure of the request pathologist, the providing information is not limited to this. For example, the providing information may be created by performing various image processes such as edge enhancement processing on the stained specimen RGB image, for example. In this case, the type of image process performed on the stained specimen RGB image may be set as the observation procedure of the pathologist.

Moreover, in the above embodiment, the diagnostic area information created by the diagnosis area information creating unit 542 has been included in the diagnostic information and distributed to the pathology diagnosis device 7 of the request pathologist. In contrast, the type of the feature amount or the spectral transmittances necessary for diagnosis for each pathologist may be set as the observation procedure. Moreover, only the values of the feature amount and the statistic amount which are set to be necessary by the request pathologist may be included in the diagnostic information and distributed to the pathology diagnosis device 7.

Moreover, in the above embodiment, in step f1 of FIG. 22, the pathologist has been retrieved based on the specimen attribute information of the diagnosis target stained specimen and the diagnostic area information of the target diagnostic area. In contrast, for example, a priority order may be set to the type of images that can be provided as the providing information, and the pathologist may be retrieved based on the diagnosis image type set as the observation procedure in the pathologist DB 6. For example, when the digitally stained image is set to be provided preferentially as the priority order of the type of images that can be provided as the providing information, the pathologist information in which the digitally stained image is set as the diagnosis image type in the dataset of the observation procedure may be first retrieved from the pathologist DB 6 and the pathologists may be narrowed down. After that, the process of selecting the request candidate pathologist from the narrowed pathologists by the similar method as the above embodiment (that is, the process of selecting the request candidate pathologists based on the diagnosis record information using the specimen attribute information of the diagnosis target stained specimen and the diagnostic area information of the target diagnostic area) may be performed. For example, if it is known in advance that a reliable diagnosis result can be expected by a diagnosis using a high value-added observation procedure like the digitally stained image, the diagnosis target stained specimen can be diagnosed by a more appropriate pathologist by retrieving and selecting a pathologist who uses the digitally stained image as the diagnosis image type.

Specifically, an optimal image is selected based on a combination of the type of images (in the example of FIG. 4, an RGB image, a dye amount image, a digitally stained image, and a pseudo-differential interference image) that can be provided as the providing information, and the diagnosis image type set in the observation procedure of the pathologist information. For example, it is assumed that the highest priority is set to the digitally stained image, and the second highest priority is set to the pseudo-differential interference image. In this case, the pathologist information in which the digitally stained image is set as the diagnosis image type in the dataset of the observation procedure is selected from the pathologist DB 6 to narrow down the pathologists, and then, the request candidate pathologist is selected using the specimen attribute information and the diagnostic area information. If the request candidate pathologist is not selected, the pathologist information in which the pseudo-differential interference image is set as the diagnosis image type is selected from the pathologist DB 6 to narrow down the pathologists, and then, the request candidate pathologist is selected using the specimen attribute information and the diagnostic area information. If the request candidate pathologist is not selected, the pathologist information in which another image (the RGB image or the dye amount image) other than the digitally stained image and the pseudo-differential interference image is set as the diagnosis image type is selected from the pathologist DB 6 to narrow down the pathologists, and then, the request candidate pathologist is selected using the specimen attribute information and the diagnostic area information.

Moreover, as for exchange of information in a remote diagnosis system in which pathologists at a remote site perform a consultation, the following method is recommended as a guideline. In the pathology diagnosis system 1 of the above embodiment, information can be exchanged by this method. Hereinafter, this method will be described briefly.

In a requesting facility that requests for a remote diagnosis and consultation, when there is a need for a remote diagnosis, the note thereof is sent to a receiving-side facility to make a reservation for the remote diagnosis via a telephone (step 1). The receiving-side facility makes preparations for the remote diagnosis on an appointment date so that devices are well organized for the diagnosis (step 2).

Moreover, an attending physician of the requesting facility delivers the key point of clinical information of a case of the requested remote diagnosis, the type and the number of remote diagnosis target samples, and the purpose of the diagnosis to the attending pathologist (receiving-side attending physician) of the receiving-side facility (step 3). After that, when the remote diagnosis target samples are actually submitted, the attending physician of the requesting facility sends an order to start preparation of specimens to the receiving-side facility over a telephone or the like (step 4).

In the receiving-side facility having received the order to start preparation of specimens in step 4, the receiving-side attending physician starts the remote diagnosis system (step 5). On the other hand, an attending examination engineer of the requesting facility prepares specimens to capture the image thereof (for example, a multiband image) and sends transmission information including the captured specimen image to the receiving-side attending physician of the receiving-side facility (step 6).

The receiving-side attending physician of the receiving-side facility having received the transmission information in step 6 displays the specimen image on a screen and makes a diagnosis while observing the image. Moreover, the receiving-side attending physician sends a request to send additional information to the attending physician of the requesting facility (step 7). Moreover, the receiving-side attending physician of the receiving-side facility directly delivers the history and the result of the diagnosis in step 7 to the attending physician of the requesting facility via a telephone or the like (step 8). Moreover, during the telephone conversation or the like, information on the diagnosis image which serves as a determining factor is presented on the screen of a computer which is synchronized and shared by the requesting facility and the receiving-side facility, and the diagnosis result is presented as character information. In this way, the history and the result of the diagnosis are reliably delivered to the attending physician of the requesting facility.

After the remote diagnosis is finished, the examination engineer of the requesting facility quickly sends the prepared specimen to the receiving-side attending physician of the receiving-side facility, who made the remote diagnosis by a method such as express delivery (step 9). Upon receiving the specimen delivered in step 9, the receiving-side attending physician of the receiving-side facility personally observes the delivered specimen using a microscope to make a diagnosis again to determine the correctness of the remote diagnosis in step 7 (step 10). When it is determined in step 10 that there was an error in the diagnosis, the note thereof is immediately transmitted to the attending physician of the requesting facility (step 11).

The history and the result of the remote diagnosis obtained in this way are stored in an appropriate electronic medium together with the entire transmission information such as the specimen image so that the stored information can be immediately reproduced if necessary (step 12). Moreover, a telepathology engineer of the requesting facility and the receiving-side attending physician of the receiving-side facility hold a face-to-face meeting periodically to share various internal and external issues on telepathology to present better operation and utilization methods (step 13).

As an example of an image format used when transmitting the multiband image to the receiving-side facility at a remote site in step 6, a spectral image system is disclosed in "Final R&D report of R&D Project of Natural Vision" (Next-generation Video Display and Transmission System) (March 31, 2006, National Institute of Information and Communications Technique (Hub Research Promotion Division). Briefly, according to this report, the spectral transmittance can also be used as well as the CIE 1931 XYZ color space defined by the ICC profile. Depending on the PCS used, the data necessary as a profile is different. For example, a case where the color space by the spectral transmittances is used as the PCS will be considered. In this case, the image data input from an input device is subjected to spectrum-based color reproduction processing, whereby color conversion processing is performed so that the image data can be output to a display device or the like.

In this color conversion processing, the spectral transmittances are estimated for each pixel, whereby spectral transmittance-based image data is generated. The spectral transmittance-based image data is device-independent color information (which is not dependent on devices). The spectral transmittance-based image data is analyzed to be a signal (data) that can be treated as the PCS. Here, when the spectral transmittances are estimated, a spectral transmittance estimation matrix is generally used. The spectral transmittance estimation matrix itself or the information necessary for calculating the spectral transmittance estimation matrix is included in an input profile which is data that correlates an image input device and the PCS.

Moreover, if the image data is image data of the PCS space, it is possible to store and transmit the image data without taking the properties of an output device into consideration. However, when the image data is stored or transmitted, the data volume may increase if the respective pixels constituting the image data possess spectral transmittance data. For example, if the respective pixels constituting the image data possess a number of data corresponding to the dimension number in the wavelength direction, it is necessary to store and transmit a number of image data obtained by multiplying the pixel count by the dimension number. Thus, in order to prevent an increase in the volume of data stored or transmitted, it is preferable that both the captured signals and the input profile be stored or transmitted as data, and a device that reads or transmits the data generate image data on the PCS space.

Reference Signs List

| 1 | PATHOLOGY DIAGNOSIS SYSTEM |
|---|---|
| 2 | VIRTUAL SLIDE MICROSCOPE |
| 21 | ELECTRIC STAGE |
| 221, 231 | MOTOR |
| 223 | XY DRIVING CONTROL UNIT |
| 233 | Z DRIVING CONTROL UNIT |
| 24 | MICROSCOPE BODY |
| 251 | COLLECTOR LENS |
| 252 | ILLUMINATION SYSTEM FILTER UNIT |
| 253 | FIELD STOP |
| 254 | APERTURE STOP |
| 255 | FOLDING MIRROR |
| 256 | CONDENSER OPTICAL ELEMENT UNIT |
| 257 | TOP LENS UNIT |
| 26 | REVOLVER |
| 27 | OBJECTIVE LENS |
| 28 | LIGHT SOURCE |
| 29 | LENS BARREL |

| 291 | BEAM SPLITTER |
|---|---|
| 30 | FILTER UNIT |
| 31 | BINOCULAR UNIT |
| 32 | TV CAMERA |
| 33 | MICROSCOPE CONTROLLER |
| 34 | TV CAMERA CONTROLLER |
| 35 | CONTROL UNIT |
| 4 | STAINED SPECIMEN DB |
| 5 | INFORMATION DISTRIBUTING DEVICE |
| 51 | INPUT UNIT |
| 52 | DISPLAY UNIT |
| 53 | COMMUNICATION UNIT |
| 54 | IMAGE PROCESSING UNIT |
| 541 | DIAGNOSTIC AREA EXTRACTION PROCESSING UNIT |
| 542 | DIAGNOSIS AREA INFORMATION CREATING UNIT |
| 543 | CANCER POTENTIAL ESTIMATING UNIT |
| 544 | PROVIDING INFORMATION CREATING UNIT |
| 545 | IMAGE MODIFICATION PROCESSING UNIT |
| 55 | STORAGE UNIT |
| 56 | CONTROL UNIT |
| 561 | PATHOLOGIST RETRIEVING UNIT |
| 562 | DIAGNOSIS REQUEST ACCEPTABILITY DETERMINING UNIT |
| 563 | PROVIDING INFORMATION DISTRIBUTION PROCESSING UNIT |
| 6 | PATHOLOGIST DB |
| 7 (7-1, 7-2, 7-3, ...) | PATHOLOGY DIAGNOSIS DEVICE |
| 8 | INFORMATION INTEGRATING DEVICE |

**Claims**

1. A diagnostic information distribution device which is configured to be communicable with multiple pathology diagnosis devices operated by different pathologists and to distribute diagnostic information to the pathology diagnosis devices, comprising:

an image acquiring unit that acquires a specimen image by imaging a diagnosis target specimen;

a diagnostic area extracting unit that extracts a diagnostic area from the specimen image;

a pathologist selecting unit that selects a request pathologist who is requested to make a diagnosis among the pathologists operating the multiple pathology diagnosis devices;

a providing information creating unit that performs image processing corresponding to a predetermined observation procedure of the request pathologist on the image data of at least the diagnostic area so as to create providing information; and

a providing information distributing unit that distributes diagnostic information including the providing information to the pathology diagnosis device of the request pathologist.

2. The diagnostic information distribution device according to claim 1, further comprising:

a feature amount calculating unit that calculates a feature amount of the diagnostic area based on at least pixel values of respective pixels constituting the diagnostic area, wherein

the providing information creating unit creates the providing information using the feature amount.

3. The diagnostic information distribution device according to claim 2, wherein

the feature amount calculating unit calculates the feature amount by extracting pixels of a predetermined component constituting the specimen from the diagnostic area, and

the providing information creating unit includes an identification image generating unit that generates an image so that a region of the predetermined component in the diagnostic area is identified.

4. The diagnostic information distribution device according to claim 2, wherein

the specimen is a stained specimen stained with a predetermined staining dye,

the image acquiring unit acquires a spectral image as the specimen image,

the feature amount calculating unit calculates the feature amount by estimating a dye amount of the staining dye at a specimen point on the corresponding specimen image for each of the pixels constituting the diagnostic area based on pixel values of the spectral image, and

the providing information creating unit includes a dye amount image generating unit that generates an image representing dye amounts at the respective pixel positions based on the dye amounts estimated for the respective pixels in the diagnostic area.

5. The diagnostic information distribution device according to claim 1, wherein

the pathologist selecting unit selects the request pathologist based on at least diagnosis record information of each of the pathologists of the pathology diagnosis devices.

6. The diagnostic information distribution device according to claim 5, further comprising

a statistic amount calculating unit that calculates a statistic amount of the diagnostic area further based on the feature amount calculated by the feature amount calculating unit, wherein

the pathologist selecting unit selects the request pathologist based on the statistic amount.

7. The diagnostic information distribution device according to claim 6, further comprising

a cancer potential estimating unit that estimates a cancer potential indicating the degree of possibility that a portion photographed in the diagnostic area is a cancer based on the statistic amount, wherein

the pathologist selecting unit selects the request pathologist based on the cancer potential.

8. The diagnostic information distribution device according to claim 5, wherein

the pathologist selecting unit selects the request pathologist further based on an observation procedure of each of the pathologists of the pathology diagnosis devices.

9. The diagnostic information distribution device according to claim 5, wherein

the pathologist selecting unit selects the request pathologist further based on schedule information of each of the pathologists of the pathology diagnosis devices.

10. The diagnostic information distribution device according to claim 2, wherein

the providing information distributing unit includes the feature amount of the diagnostic area in the diagnostic information and distributes the diagnostic information to the pathology diagnosis device of the request pathologist.

**11.** The diagnostic information distribution device according to claim 6, wherein
the providing information distributing unit includes the statistic amount of the diagnostic area in the diagnostic information and distributes the diagnostic information to the pathology diagnosis device of the request pathologist.

**12.** A pathology diagnosis system in which a diagnostic information distribution device and multiple pathology diagnosis devices operated by different pathologists are connected via a network, wherein
the diagnostic information distribution device comprises:

an image acquiring unit that acquires a specimen image by imaging a diagnosis target specimen;
a diagnostic area extracting unit that extracts a diagnostic area from the specimen image;
a pathologist selecting unit that selects a request pathologist who is requested to make a diagnosis among the pathologists operating the multiple pathology diagnosis devices;
a providing information creating unit that performs image processing corresponding to a predetermined observation procedure of the request pathologist on the image data of at least the diagnostic area so as to create providing information; and
a providing information distributing unit that distributes diagnostic information including at least the providing information to the pathology diagnosis device of the request pathologist, and
wherein the pathology diagnosis device includes a display processing unit that displays the providing information on a display unit.

**13.** The pathology diagnosis system according to claim 12, further comprising
a pathologist storage unit that is connected to at least the diagnostic information distribution device via the network, wherein
the pathologist storage unit stores pathologist information including at least one of observation procedure information, diagnosis record information, and schedule information of the corresponding pathologist.

**14.** The pathology diagnosis system according to claim 12, wherein
the diagnostic information distribution device is connected to an observing unit that observes the specimen using a microscope,
the observing unit captures each portion of the specimen while moving the specimen relative to an objective lens in a plane perpendicular to an optical axis of the objective lens to acquire multiple specimen images, and
the observing unit includes a specimen image generating unit that generates one specimen image by combining the multiple specimen images.

**Amended claims under Art. 19.1 PCT**

**1.** [Amended] A diagnostic information distribution device which is configured to be communicable with multiple pathology diagnosis devices and to distribute diagnostic information to the pathology diagnosis devices, comprising:

an image acquiring unit that acquires a specimen image by imaging a diagnosis target specimen;
a diagnostic area extracting unit that extracts a diagnostic area from the specimen image;
a providing information creating unit that modifies the image data of at least the diagnostic area into an image of an image type corresponding to an observation procedure correlated with a request pathologist who is requested to make a diagnosis so as to create providing information; and
a providing information distributing unit that distributes diagnostic information including the providing information to the pathology diagnosis device of the request pathologist.

**2.** The diagnostic information distribution device according to claim 1, further comprising:

a feature amount calculating unit that calculates a feature amount of the diagnostic area based on at least pixel values of respective pixels constituting the diagnostic area, wherein
the providing information creating unit creates the providing information using the feature amount.

**3.** The diagnostic information distribution device according to claim 2, wherein
the feature amount calculating unit calculates the feature amount by extracting pixels of a predetermined component constituting the specimen from the diagnostic area, and
the providing information creating unit includes an identification image generating unit that generates an image so that a region of the predetermined component in the diagnostic area is identified.

**4.** The diagnostic information distribution device according to claim 2, wherein
the specimen is a stained specimen stained with a predetermined staining dye,
the image acquiring unit acquires a spectral image as the specimen image,
the feature amount calculating unit calculates the feature amount by estimating a dye amount of the staining dye at a specimen point on the corresponding specimen image for each of the pixels constituting the diagnostic area based on pixel values of the spectral image, and
the providing information creating unit includes a dye amount image generating unit that generates an image representing dye amounts at the respective pixel positions based on the dye amounts estimated for the respective pixels in the diagnostic area.

**5.** [Amended] The diagnostic information distribution device according to claim 1, further comprising:

a pathologist selecting unit that selects the request pathologist who is requested to make a diagnosis from multiple pathologists, wherein
the pathologist selecting unit selects the request pathologist based on at least diagnosis record information of each of the pathologists of the pathology diagnosis devices.

**6.** The diagnostic information distribution device according to claim 5, further comprising
a statistic amount calculating unit that calculates a statistic amount of the diagnostic area further based on the feature amount calculated by the feature amount calculating unit, wherein
the pathologist selecting unit selects the request pathologist based on the statistic amount.

**7.** The diagnostic information distribution device according to claim 6, further comprising
a cancer potential estimating unit that estimates a cancer potential indicating the degree of possibility that a portion photographed in the diagnostic area is a cancer based on the statistic amount, wherein
the pathologist selecting unit selects the request pathologist based on the cancer potential.

**8.** The diagnostic information distribution device according to claim 5, wherein
the pathologist selecting unit selects the request pathologist further based on an observation procedure of each of the pathologists of the pathology diagnosis devices.

**9.** The diagnostic information distribution device according to claim 5, wherein
the pathologist selecting unit selects the request pathologist further based on schedule information of each of the pathologists of the pathology diagnosis devices.

**10.** The diagnostic information distribution device according to claim 2, wherein
the providing information distributing unit includes the feature amount of the diagnostic area in the diagnostic information and distributes the diagnostic information to the pathology diagnosis device of the request pathologist.

**11.** The diagnostic information distribution device according to claim 6, wherein
the providing information distributing unit includes the statistic amount of the diagnostic area in the diagnostic information and distributes the diagnostic information to the pathology diagnosis device of the request pathologist.

**12.** [Amended] A pathology diagnosis system in which a diagnostic information distribution device and multiple pathology diagnosis devices are connected via a network, wherein
the diagnostic information distribution device comprises:

an image acquiring unit that acquires a specimen image by imaging a diagnosis target specimen;
a diagnostic area extracting unit that extracts a diagnostic area from the specimen image;
a providing information creating unit that modifies the image data of at least the diagnostic area into an image of an image type corresponding to an observation procedure correlated with a request pathologist who is requested to make a diagnosis so as to create providing information; and
a providing information distributing unit that distributes diagnostic information including the providing information to the pathology diagnosis device of the request pathologist, and
wherein the pathology diagnosis device includes a dis play processing unit that displays the providing information on a display unit.

EP 2 544 141 A1

**13.** The pathology diagnosis system according to claim 12, further comprising
a pathologist storage unit that is connected to at least the diagnostic information distribution device via the network, wherein
the pathologist storage unit stores pathologist information including at least one of observation procedure information, diagnosis record information, and schedule information of the corresponding pathologist.

**14.** The pathology diagnosis system according to claim 12, wherein
the diagnostic information distribution device is connected to an observing unit that observes the specimen using a microscope,
the observing unit captures each portion of the specimen while moving the specimen relative to an objective lens tin a plane perpendicular to an optical axis of the objective lens to acquire multiple specimen images, and
the observing unit includes a specimen image generating unit that generates one specimen image by combining the multiple specimen images.

In claim 1, the expression that multiple pathology diagnosis devices are operated by different pathologists has been deleted therefrom. Moreover, the pathologist selecting means has been deleted from the claim elements. Moreover, an amendment has been made such that the providing information creating means modifies the image data of at least the diagnostic area into an image of an image type corresponding to an observation procedure correlated with a request pathologist who is requested to make a diagnosis so as to create providing information (see paragraphs 0020, 0045, 0149, 0169, and 0170).

In claim 5, an amendment has been made such that the diagnostic information distribution device further comprises a pathologist selecting means that selects the request pathologist who is requested to make a diagnosis from multiple pathologists (see paragraph 0042).

In claim 12, the same amendments as claim 1 have been made.

# FIG.1

# FIG.2

EP 2 544 141 A1

# FIG.3

5

| COMMUNICATION UNIT | ~53 |

56~

**CONTROL UNIT**

561~ | PATHOLOGIST RETRIEVING UNIT |

562~ | DIAGNOSIS REQUEST ACCEPTABILITY DETERMINING UNIT |

563~ | PROVIDING INFORMATION DISTRIBUTION PROCESSING UNIT |

| INPUT UNIT | ~51 |

| DISPLAY UNIT | ~52 |

| STORAGE UNIT | ~55 |

**IMAGE PROCESSING UNIT** ~54

| DIAGNOSTIC AREA EXTRACTION PROCESSING UNIT | ~541 |

| DIAGNOSIS AREA INFORMATION CREATING UNIT | ~542 |

| CANCER POTENTIAL ESTIMATING UNIT | ~543 |

| PROVIDING INFORMATION CREATING UNIT | ~544 |

| IMAGE MODIFICATION PROCESSING UNIT | ~545 |

# FIG.4

VIRTUAL SLIDE MICROSCOPE `2`

a1

D1
- SPECIMEN ATTRIBUTE INFORMATION
  - ORGAN TYPE
  - TARGET TISSUE TYPE
  - STAINING METHOD
  - PATIENT INFORMATION
- STAINED SPECIMEN IMAGE

STAINED SPECIMEN DB `4`

a3

D9
- DIAGNOSTIC AREA INFORMATION
- PROVIDING INFORMATION

a9

D3
- SPECIMEN ATTRIBUTE INFORMATION
- STAINED SPECIMEN IMAGE

D17
- FINAL DIAGNOSIS RESULT INFORMATION

a17

a15

D15
- SPECIMEN ATTRIBUTE INFORMATION
- STAINED SPECIMEN IMAGE
- DIAGNOSTIC AREA INFORMATION
- PROVIDING INFORMATION

INFORMATION DISTRIBUTING DEVICE `5`

a7

D5
- PATHOLOGIST INFORMATION
  - EXPERIENCE, SPECIALIZED FIELD
  - PAST CASE (ORGAN, TISSUE, ···)
  - OBSERVATION PROCEDURE
  - SCHEDULE

a5

PATHOLOGIST DB `6`

D13
- DIAGNOSIS CONTENT INFORMATION

D7
- SPECIMEN ATTRIBUTE INFORMATION
- STAINED SPECIMEN IMAGE
- DIAGNOSTIC AREA INFORMATION
  - POSITIONAL INFORMATION, CENTRAL POSITION, ···
  - FEATURE AMOUNT
    - DYE AMOUNT
    - COLOR INFORMATION CORRECTION COEFFICIENT
    - COMPONENT INFORMATION (NUCLEUS, FIBER, BLOOD VESSEL, ···)
  - STATISTIC AMOUNT
    - NUCLEUS STATISTIC AMOUNT
    - FIBER STATISTIC AMOUNT
    - BLOOD VESSEL STATISTIC AMOUNT
  - CANCER POTENTIAL
    - GRADE
- PROVIDING INFORMATION
  - RGB IMAGE/DYE AMOUNT IMAGE/ DIGITALLY STAINED IMAGE/PSEUDO-DIFFERENTIAL INTERFERENCE IMAGE/···

PATHOLOGY DIAGNOSIS DEVICE `7`

a13    a11

INFORMATION INTEGRATING DEVICE `8`

D11
- DIAGNOSIS REPORT INFORMATION
  - OPINION
  - DIAGNOSIS RESULT

EP 2 544 141 A1

# FIG.5

VIRTUAL SLIDE MICROSCOPE

( START )

↓

b1
ACQUIRE SPECIMEN ATTRIBUTE
INFORMATION

↓

b3
VIRTUAL SLIDE IMAGE
GENERATING PROCESS

↓

b5
TRANSMIT SPECIMEN ATTRIBUTE
INFORMATION AND STAINED
SPECIMEN IMAGE AND SEND WRITE
REQUEST

↓

( END )

→

4
STAINED
SPECIMEN DB

⟨ STORE STAINED
SPECIMEN INFORMATION
AND STAINED SPECIMEN
IMAGE AS STAINED
SPECIMEN INFORMATION ⟩

# FIG.6

VIRTUAL SLIDE IMAGE
GENERATING PROCESS

SWITCH TO LOW-MAGNIFICATION OBJECTIVE LENS — c1

ACQUIRE LOW-RESOLUTION IMAGE — c3

GENERATE ENTIRE SLIDE SPECIMEN IMAGE — c5

SWITCH TO HIGH-MAGNIFICATION OBJECTIVE LENS — c7

DETERMINE SPECIMEN AREA — c9

EXTRACT FOCUS POSITION AT WHICH IN-FOCUS POSITION IS MEASURED — c11

MEASURE IN-FOCUS POSITION AT FOCUS POSITION — c13

CREATE FOCUS MAP — c15

ACQUIRE HIGH-RESOLUTION IMAGE OF SPECIMEN IMAGE FOR EACH SMALL SECTION OF SPECIMEN AREA IMAGE — c17

GENERATE VIRTUAL SLIDE IMAGE — c19

COMPOSE STAINED SPECIMEN RGB IMAGE — c21

RETURN

# FIG.7

# FIG.8

◩ FOCUS POSITION

# FIG.9

| ARRANGEMENT NUMBER | | | ELECTRIC STAGE POSITION | | |
|---|---|---|---|---|---|
| x | y | z | X | Y | Z |
| 1 | 1 | – | $X_{11}$ | $Y_{11}$ | $Z_{11}$ |
| 1 | 2 | – | $X_{12}$ | $Y_{12}$ | $Z_{12}$ |
| ⋮ | ⋮ | ⋮ | ⋮ | ⋮ | ⋮ |

FIG.10

EP 2 544 141 A1

# FIG.11

INFORMATION DISTRIBUTING
DEVICE

( DIAGNOSTIC AREA
INFORMATION CREATING
PROCESS )

e1

EXTRACT DIAGNOSTIC AREA

e3

CALCULATE FEATURE AMOUNT OF
EXTRACTED DIAGNOSTIC AREA

e5

CALCULATE STATISTIC AMOUNT OF
DIAGNOSTIC AREA BASED ON
CALCULATED FEATURE AMOUNT

e7

ESTIMATE CANCER POTENTIAL

e9

IS
CANCER POTENTIAL
"LEVEL 4"?

NO

YES

e11

DETERMINE GRADE

e13

TRANSMIT DIAGNOSTIC AREA
INFORMATION

RETURN

4

STAINED
SPECIMEN DB

<UPDATE STAINED
SPECIMEN INFORMATION>

# FIG.12

MK11

EXTRACTION OF DIAGNOSTIC AREA

W11

MK15

MK13

SELECTION MODE

◉ square          ○ ellipse
○ auto square     ○ auto ellipse
○ auto picker
○ manual

Block size:   3  pixel

MEMO ────── B11

RETRY        OK

RB11          IB11    M11          B13      B15

# FIG.13

# FIG.14

## FIG.15

HOLE

HOLE

BLOOD VESSEL

## FIG.16

E31

G3

L3

Ek3

E3

# FIG.17

E41

d$_{1,2}$

E42

g$_2$(x$_2$, y$_2$)

(NL = 2)

(NL = 1)

(NL = 3)

g$_1$(x$_1$, y$_1$)

E43

# FIG.18

(a)

E45

P45

(b)

P451

L451

E45

P453

P452

P45

(c)

E45

P45

# FIG.19

P5

P53

L51

L53

# FIG.20

L3

E33

E3

# FIG.21

| | DENSITY LEVEL: 1 | DENSITY LEVEL: 2 | DENSITY LEVEL: 3 |
|---|---|---|---|
| $PS \ll Th_{PS}$ | LEVEL = 4 | LEVEL = 3 | LEVEL = 2 |
| $PS < Th_{PS}$ | LEVEL = 4 | LEVEL = 3 or 2 | LEVEL = 2 |
| $PS \geq Th_{PS}$ | LEVEL = 2 | LEVEL = 2 or 1 | LEVEL = 1 |

# FIG.22

```
INFORMATION                                                    PATHOLOGY
DISTRIBUTING DEVICE                                          DIAGNOSIS DEVICE
```

PATHOLOGIST
SELECTING PROCESS

START

↓ ⌐f1

RETRIEVE PATHOLOGIST BASED ON
DIAGNOSTIC AREA INFORMATION or
THE LIKE AND ACQUIRE
PATHOLOGIST REGISTRATION
INFORMATION

⌐6
PATHOLOGIST
DB

↓ ⌐f3

SELECT REQUEST CANDIDATE
PATHOLOGIST BASED ON SCHEDULE

↓ ⌐f5

SEND REQUEST FOR REPLY TO
DIAGNOSIS REQUEST TO
PATHOLOGY DIAGNOSIS DEVICE OF
REQUEST CANDIDATE PATHOLOGIST

IS
ACCEPTABILITY
INPUT? ⌐g1 — NO

↓ YES ⌐g3

TRANSMIT
ACCEPTABILITY
INFORMATION

NO ← IS ACCEPT-
ABILITY INFORMATION
RECEIVED? ⌐f7

↓ YES

NO• ← HAS
THE NUMBER OF
PATHOLOGISTS HAVING
ACCEPTED REQUEST REACHED
UPPER LIMIT
COUNT? ⌐f9

↓ YES ⌐f11

DETERMINE REQUEST CANDIDATE
PATHOLOGIST

↓ ⌐f13

MODIFY STAINED SPECIMEN IMAGE
IN ACCORDANCE WITH
OBSERVATION PROCEDURE OF
REQUEST CANDIDATE PATHOLOGIST
TO CREATE PROVIDING
INFORMATION

↓ ⌐f15

TRANSMIT PROVIDING INFORMATION

⌐4
STAINED
SPECIMEN DB

RETURN

END

<UPDATE STAINED
SPECIMEN
INFORMATION>

# FIG.23

| PATHOL-OGIST ID | NAME | POSI-TION | NETWORK ID | MAIL ADDRESS | EXPE-RIENCE/ SPECIAL-IZED FIELD | OBSER-VATION PROCE-DURE | DIAGNOSED ORGAN/ TISSUE | GRADE/ RARE CASE NUMBER | SCHEDULE |
|---|---|---|---|---|---|---|---|---|---|
| 1 | ○○○○ | HOSPI-TAL A | XXXX | XX@XXXX | XXXXX | DATASET A-01 | DATASET B-01 | DATASET C-01 | DATASET D-01 |
| 2 | △△△△ | HOSPI-TAL A | XXXX | XX@XXXX | XXXXX | DATASET A-02 | DATASET B-02 | DATASET C-02 | DATASET D-02 |
| 3 | □□□□ | HOSPI-TAL A | XXXX | XX@XXXX | XXXXX | DATASET A-03 | DATASET B-03 | DATASET C-03 | DATASET D-03 |
| 4 | ◎◎◎◎ | HOSPI-TAL A | XXXX | XX@XXXX | XXXXX | DATASET A-04 | DATASET B-04 | DATASET C-04 | DATASET D-04 |
| 5 | ◇◇◇◇ | HOSPI-TAL A | XXXX | XX@XXXX | XXXXX | DATASET A-05 | DATASET B-05 | DATASET C-05 | DATASET D-05 |
| ⋮ | ⋮ | ⋮ | ⋮ | ⋮ | ⋮ | ⋮ | ⋮ | ⋮ | ⋮ |

EP 2 544 141 A1

# FIG.24-1

| ORGAN | TISSUE | CASE RECORD | PERCENTAGE |
|---|---|---|---|
| ENDOCERVIX | SQUAMOUS EPITHELIUM | 30 | 0.38 |
| ENDOCERVIX | MUCOUS GLAND | 20 | 0.25 |
| STOMACH | MUCOUS EPITHELIUM | 3 | 0.03 |
| STOMACH | LEIOMY-OSARCOMA | 10 | 0.12 |
| PROSTATE | SQUAMOUS EPITHELIUM | 10 | 0.12 |
| PROSTATE | BASAL CELL | 5 | 0.06 |
| ⋮ | ⋮ | ⋮ | ⋮ |

~ L61

# FIG.24-2

| ORGAN | TISSUE | CASE RECORD | PERCENTAGE |
|---|---|---|---|
| ENDOCERVIX | SQUAMOUS EPITHELIUM | 20 | 0.25 |
| ENDOCERVIX | MUCOUS GLAND | 3 | 0.03 |
| STOMACH | MUCOUS EPITHELIUM | 10 | 0.12 |
| STOMACH | LEIOMY-OSARCOMA | 10 | 0.12 |
| PROSTATE | SQUAMOUS EPITHELIUM | 5 | 0.06 |
| PROSTATE | BASAL CELL | 5 | 0.06 |
| ⋮ | ⋮ | ⋮ | ⋮ |

~ L62

# FIG.25

| ORGAN TYPE | | ENDOCERVIX |
|---|---|---|
| TARGET TISSUE TYPE | | SQUAMOUS EPITHELIUM |
| STAINING METHOD | | H&E STAINING |
| ⋮ | | ⋮ |
| NUCLEUS STATISTIC AMOUNT | INTER-NUCLEUS DISTANCE | XXX |
| | NUCLEAR ATYPICALITY | XXX |
| | ⋮ | ⋮ |
| ⋮ | | ⋮ |
| BLOOD VESSEL STATISTIC AMOUNT | DEGREE OF SHAPE IRREGULARITY | XXX |
| | ⋮ | ⋮ |
| ⋮ | | ⋮ |
| CANCER POTENTIAL | | LEVEL 4 |
| ESTIMATED GRADE | | II |
| NUCLEAR DENSITY LEVEL | | LEVEL 2 |
| URGENCY LEVEL | | 1 |

# FIG.26

| NEW MAIL | REPLY ▽ | REPLY TO ALL | SEND ▽ | DELETE | FOLLOW-UP ▽ |
|---|---|---|---|---|---|

| FOLDER ▽ | ADD COPY ▽ | CHART ▽ | DISPLAY ▽ | TOOL ▽ |
|---|---|---|---|---|

XX@XXXX

2009/XX/XX

| To | XX@XXXX |
|---|---|
| cc | |
| bcc | |
| Subject | DIAGNOSIS REQUEST |

DEAR DR. XX TO HOSPITAL A

A DIAGNOSIS REQUEST HAS BEEN RECEIVED.
PLEASE REVIEW THE CONTENTS AND
PRESS "ACCEPT" BUTTON OR "REJECT" BUTTON.

**REQUESTED CONTENTS**
ORGAN: ENDOCERVIX
TISSUE: SQUAMOUS EPITHELIUM

⋮

DUE DATE: (MMDD)

| ACCEPT | REJECT |
|---|---|

B61    B63

# FIG.27

| NEW MAIL | REPLY ▽ | REPLY TO ALL | SEND ▽ | DELETE | FOLLOW-UP ▽ |
|---|---|---|---|---|---|

| FOLDER ▽ | ADD COPY ▽ | CHART ▽ | DISPLAY ▽ | TOOL ▽ |
|---|---|---|---|---|

XX@XXXX

2009/XX/XX

| | |
|---|---|
| To | XX@XXXX |
| cc | |
| bcc | |
| Subject | NOTIFICATION OF ACCESSIBILITY |

DEAR DR. XX TO HOSPITAL A

THANK YOU FOR ACCEPTING THE REQUEST.
PLEASE BE INFORMED THAT THE SERVER IS CURRENTLY ACCESSIBLE.

# FIG.28

| NEW MAIL | REPLY ▽ | REPLY TO ALL | SEND▽ | DELETE | FOLLOW-UP ▽ |

| FOLDER ▽ | ADD COPY ▽ | CHART ▽ | DISPLAY ▽ | TOOL ▽ |

XX@XXXX

2009/XX/XX

To | XX@XXXX

cc |

bcc |

Subject | NOTIFICATION OF ARRIVAL OF UPPER LIMIT COUNT

DEAR DR. YY TO HOSPITAL B

YOU HAVE RECEIVED THE DIAGNOSIS REQUEST, BUT PLEASE BE INFORMED THAT THE UPPER LIMIT COUNT HAS REACHED.

WE APPRECIATE YOUR CONTINUED COOPERATION.

# FIG.29

# FIG.30

FIG.31

# FIG.32

# FIG.33

| CREATION OF DIAGNOSIS REPORT |||||||||
|---|---|---|---|---|---|---|---|---|

| PATHOLOGY NUMBER | XXXX | | | | ORDER NUMBER | | XXXX | |
|---|---|---|---|---|---|---|---|---|
| RECEIVED DATE | MAY 13, 2011 | | | | HARVESTED DATE | | MAY 12, 2011 | |
| PATIENT ID | XXX | NAME | XXX | GEN-DER | FE-MALE | BIRTH DATE | JANUARY 30, YYYY | AGE | XX YEARS, 3 MONTHS |
| IN/OUT-PATIENT | IN-PATIENT | DEPART-MENT | MAMMARY GLAND AND THYROID | WARD | | XX | REQUESTING PHYSICIAN | XXXX |
| | | | | | | | | |
| CLINICAL DISEASE | PAPILLARY THYROID CANCER IS SUSPECTED/ MEDIAN CERVICAL CYST | | | | | | | |
| CLINICAL OPINION | POSITIVE IN THE LEFT LOBE OF THE THYROID GLAND OF 3 cm LUMP ABC | | | | | | | |
| COMMENT | | | | | | | | |
| EXAMINED ORGAN | TOTAL THYROIDECTOMY ··· | | | | | | | |
| REPORTING DATE | MAY 20, 2011 | | REPORTER | | XXXX | | REPORTER | |

W91

PATHOLOGICAL OPINION

(1) XXXX···
(2) XXXX···

DIAGNOSIS RESULT

IB91

| DISEASE NAME | |
|---|---|
| GRADE | |

IB93

FINALIZE — B91

SUSPEND — B93

W93

## INTERNATIONAL SEARCH REPORT

| International application No. |
| --- |
| PCT/JP2011/054657 |

A.   CLASSIFICATION OF SUBJECT MATTER
*G06Q50/00*(2006.01)i, *G01N21/27*(2006.01)i, *G01N33/483*(2006.01)i

According to International Patent Classification (IPC) or to both national classification and IPC

B.   FIELDS SEARCHED

Minimum documentation searched (classification system followed by classification symbols)
G06Q50/00, G01N21/27, G01N33/483

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| Y | JP 2005-165648 A  (Canon Inc.), 23 June 2005 (23.06.2005), paragraph [0009] (Family: none) | 1-14 |
| Y | JP 2002-073615 A  (Olympus Optical Co., Ltd.), 12 March 2002 (12.03.2002), paragraph [0108] (Family: none) | 1-14 |
| Y | Nagaaki OYAMA, "Multi-spectral Gazo o Mochiita Shindan Shien Gijutsu no Kaihatsu", [online], Kagaku Gijutsu Shinko Choseihi Seika Hokokusho, 2004.06, [retrieval date 11 May 2011 (11.05.2011)], Internet <http://scfdb.tokyo.jst.go.jp/pdf/20011790/2003/200117902003rr.pdf> | 1-14 |

| ☒ | Further documents are listed in the continuation of Box C. | ☐ | See patent family annex. |
| --- | --- | --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 13 May, 2011 (13.05.11) | 24 May, 2011 (24.05.11) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/054657

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-196533 A  (Canon Inc.),<br>21 July 2005 (21.07.2005),<br>entire text; all drawings<br>(Family: none) | 1-14 |
| A | JP 2005-092706 A  (Canon Inc.),<br>07 April 2005 (07.04.2005),<br>entire text; all drawings<br>(Family: none) | 1-14 |
| A | JP 2002-132958 A  (JB Kabushiki Kaisha),<br>10 May 2002 (10.05.2002),<br>paragraph [0007]<br>(Family: none) | 1-14 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11195077 A
- JP 7120324 A
- JP 9281405 A

**Non-patent literature cited in the description**

- Color Correction of Pathological Images Based on Dye Amount Quantification. *OPTICAL REVIEW,* 2005, vol. 12 (4), 293-300
- Development of support systems for pathology using spectral transmittance - The quantification method of stain conditions. *Proceedings of SPIE - Image Processing,* vol. 4684, 1516-1523
- Final R&D report of R&D Project of Natural Vision. *Next-generation Video Display and Transmission System,* 31 March 2006